# EUROPEAN PATENT SPECIFICATION

(11) **EP 0 092 182 B1**
(45) Date of publication and mention of the grant of the patent: **12.10.1994**
(21) Application number: 83103629.8
(22) Date of filing: 14.04.1983
(51) Int. Cl.: C12N 15/58, C12N 9/72, C12N 1/00, A61K 37/54, C12R 1/19, C12R 1/865, C12R 1/91

(54) **Preparation of functional human urokinase polypeptides**
Herstellung von funktionellen menschlichen Urokinasepolypeptiden
Préparation de polypeptides fonctionnelles d'urokinase humaine

(30) Priority: 15.04.1982 US 368773; 14.03.1983 US 474930
(43) Date of publication of application: 26.10.1983
(60) Divisional application: 94104777.1
(73) Proprietor: GENENTECH, INC., South San Francisco California 94080 (US)
(72) Inventor: Heyneker, Herbert Louis, Burlingame, CA 94010 (US); Holmes, William Evans, Pacifica, CA 94044 (US); Vehar, Gordon Alan, San Carlos, CA 94070 (US)
(74) Representative: Goldin, Douglas Michael

(56) References cited:
- EP-A- 0 037 687
- EP-A- 0 049 619
- EP-B- 0 040 238
- US-A- 4 321 363
- CHEMICAL ABSTRACTS, vol. 97, no. 13, 27th September 1982, page 143, no.104934b, Columbus, Ohio, USA, P.P. Hung
- CHEMICAL ABSTRACTS, vol. 96, no. 5, 1st February 1982, page 145, no. 29397c, Columbus, Ohio, USA, A. Bollen et al.
- Hoppe-Seyler's Z. Physiol. Chem. (Feb. 1982) 363, 133-141
- PNAS USA 78 (1981) pp. 3313-3317
- Verhandlungsbericht der 25. Tagung der dt. Arbeitsgemeinschaft für Blutgerinnungsforschung, Stuttgart 1981, pp. 367-370
- The Journal of Biol. Chem., Vol. 257, No. 6, (March 1982), pp. 3276-3283
- Archives of Biochemistry and Biophysics, Vol. 220, No. 1 (1983) pp. 31-38

## Description

### Field of the Invention

The present invention relates to human urokinase polypeptide, to novel forms and compositions thereof and particularly to means and methods for the preparation in vitro of functional polypeptide species of human urokinase.

The present invention is based in part on the discovery of the DNA sequence and deduced amino acid sequence of native urokinase as well as associated portions of the urokinase molecule found to be the functional bioactive moieties. This discovery enabled the production of urokinase polypeptide in various forms via the application of recombinant DNA technology, in turn enabling the production of sufficient quality and quantity of materials with which to conduct requisite biological testing identifying the biologically functional, hence useful moieties of the molecule. Having determined such, it was possible to tailor-make functional species of urokinase polypeptide via genetic manipulation and in vitro processing, arriving efficiently at hitherto unobtainable commercially efficacious amounts of active polypeptide products. This invention is directed to these associated embodiments in all respects.

The publications and other materials hereof used to illuminate the background of the invention, and in particular cases, to provide additional details concerning its practice are incorporated herein by reference, and for convenience, are numerically referenced in the following text and respectively grouped in the appended bibliography.

### Background of the Invention

### A. Human Urokinase

The fibrinolytic system is in a dynamic equilibrium with the coagulation system, maintaining an intact, patent vascular bed. The coagulation system deposits fibrin as a matrix serving to restore a hemostatic condition. The fibrinolytic system removes the fibrin network after the hemostatic condition is achieved. The fibrinolytic process is brought about by the proteolytic enzyme plasmin that is generated from a plasma protein precursor plasminogen. Plasminogen is converted to plasmin through activation by an activator.

Urokinase is one such activator. It and another activator, streptokinase, are currently commercially available. Both are indicated for the treatment of acute vascular diseases such as myocardial infarct, stroke, pulmonary embolism, deep vein thrombosis, peripheral arterial occlusion and other venous thromboses. Collectively, these diseases account for major health hazards and risks.

The underlying etiological basis for these diseases points to either a partial, or in severe cases, total occlusion of a blood vessel by a blood clot -- thrombus or thromboembolus. Traditional anticoagulant therapy, as with heparin and coumarin, does nothing to directly enhance dissolution of thrombi or thromboemboli. Streptokinase and urokinase have enjoyed practical and effective use as thrombolytic agents. Until now, however, each has suffered from severe limitations. Neither has demonstrated a high affinity for fibrin; consequently, both activate circulating and fibrin-bound plasminogen relatively indiscriminately. The plasmin formed in circulating blood is neutralized rather quickly and lost for useful thrombolysis. Residual plasmin will degrade several clotting factor proteins, for example, fibrinogen, Factor V and Factor VIII, causing a hemorrhagic potential. In addition, streptokinase is strongly antigenic and patients with high antibody titers respond inefficiently to treatment and cannot remain on continuous treatment. Urokinase therapy is expensive, owing to its involved isolation from human urine or tissue culture, and it therefore is not generally accepted in clinical practice. Urokinase has been the subject of numerous investigations -- See, for example, references 1-9. Presently available urokinase, as defined, is isolated from human urine or tissue culture, e.g. kidney cells (9A,9B).

The urokinase molecule exists in several biologically active forms - high molecular weight (ca. 54000 daltons) and low molecular weight (ca. 33000 daltons), each composed of single chain or two chain material. The low molecular weight form is derived from the high molecular weight form by enzymatic cleavage. Biologically active material contains the so-called serine protease portion linked, in active form, to a second chain via a disulfide bond. Any activity ascribed to the high molecular weight material is believed to be due to the similar presence of these two connected chains, the strategic disulfide bond and interruption in the sequence doubtless being located in the serine protease portion of the overall molecules (See Figure A). In any event, until the present invention, the identity, and hence function, of the ca. 21000 dalton residue was unknown and the assignment of activity to one or another of the known moieties of urokinase was not uncontrovertedly possible.

Recently, there was a report of another form of urokinase peptide having low, but specific activity (10, 10A). It was speculated that this material corresponds to native urokinase, a preform of the previously isolated active species described above, most probably consisting of a single chain.

Previous attempts to clone the requisite gene for urokinase with attendant hopes of attaining expression in a microbial host were not believed successful (11, 11A). See also (6).

It was perceived that the application of recombinant DNA and associated technologies, after all, would be a most effective way of providing the requisite large quantities of high quality, bioactive human urokinase polypeptide essentially free of other human protein, and derivatives thereof that retain functional bioactivity, thus admitting of the use of such materials clinically in the treatment of various vascular conditions or diseases.

### B. Recombinant DNA/Protein Biochemistry Technology

Recombinant DNA technology has reached the age of some sophistication. Molecular biologists are able to recombine various DNA sequences with some facility, creating new DNA entities capable of producing copious amounts of exogenous protein product in transformed microbes and cell cultures. The general means and methods are in hand for the in vitro ligation of various blunt ended or "sticky" ended fragments of DNA, producing potent expression vehicles useful in transforming particular organisms, thus directing their efficient synthesis of desired exogenous product. However, on an individual product basis, the pathway remains somewhat tortuous and the science has not advanced to a stage where regular predictions of success can be made. Indeed, those who portend successful results without the underlying experimental basis, do so with considerable risk of inoperability.

DNA recombination of the essential elements, i.e., an origin of replication, one or more phenotypic selection characteristics, an expression promoter, heterologous gene insert and remainder vector, generally is performed outside the host cell. The resulting recombinant replicable expression vehicle, or plasmid, is introduced into cells by transformation and large quantities of the recombinant vehicle obtained by growing the transformant. Where the gene is properly inserted with reference to portions which govern the transcription and translation of the encoded DNA message, the resulting expression vehicle is useful to actually produce the polypeptide sequence for which the inserted gene codes, a process referred to as expression. The resulting product may be obtained by lysing, if necessary, the host cell, in microbial systems, and recovering the product by appropriate purification from other proteins.

In practice, the use of recombinant DNA technology can express entirely heterologous polypeptides--so-called direct expression--or alternatively may express a heterologous polypeptide fused to a portion of the amino acid sequence of a homologous polypeptide. In the latter cases, the intended bioactive product is sometimes rendered bioinactive within the fused, homologous/heterologous polypeptide until it is cleaved in an extracellular environment. See references (12) and (13).

Similarly, the art of cell or tissue cultures for studying genetics and cell physiology is well established. Means and methods are in hand for maintaining permanent cell lines, prepared by successive serial transfers from isolate normal cells. For use in research, such cell lines are maintained on a solid support in liquid medium, or by growth in suspension containing support nutriments. Scale-up for large preparations seems to pose only mechanical problems. For further background, attention is directed to references (14) and (15).

Likewise, protein biochemistry is a useful, indeed necessary, adjunct in biotechnology. Cells producing the desired protein also produce hundreds of other proteins, endogenous products of the cell's metabolism. These contaminating proteins, as well as other compounds, if not removed from the desired protein, would prove toxic if administered to an animal or human in the course of therapeutic treatment with desired protein. Hence, the techniques of protein biochemistry come to bear, allowing the design of separation procedures suitable for the particular system under consideration and providing a homogeneous product safe for intended use. Protein biochemistry also proves the identity of the desired product characterizing it and ensuring that the cells have produced it faithfully with no alterations or mutations. This branch of science is also involved in the design of bioassays, stability studies and other procedures necessary to apply before successful clinical studies and marketing can take place.

### Summary of the Invention

The present invention is based upon the discovery that recombinant DNA/protein biochemistry technology can be used to successfully produce human urokinase in the form of biologically functional, tailored species. This invention provides active urokinase protein suitable for use in the prophylactic or therapeutic treatment of human beings for various vascular conditions or diseases. Each of its forms includes the bioactive moiety, that is the enzymatic portion of native material believed to reside in the serine protease portion. In accordance with this invention, a series of urokinase active products can be prepared in a form available for in vitro processing to result in bioactive product. This invention also provides the means and methods for producing full length native urokinase polypeptide molecules in bioactivatable form, having the potential added advantage of specific affinity for fibrin not demonstrated until now with any urokinase product isolated from natural sources. Thus provided is human urokinase polypeptide product having the potential new property of specific activity toward tangible, extant thrombi. The products being produced by a host cell harboring recombinant DNA encoding respective product entity, the facilities are now at hand to produce human urokinase polypeptide in forms exhibiting enhanced biologically significant properties.

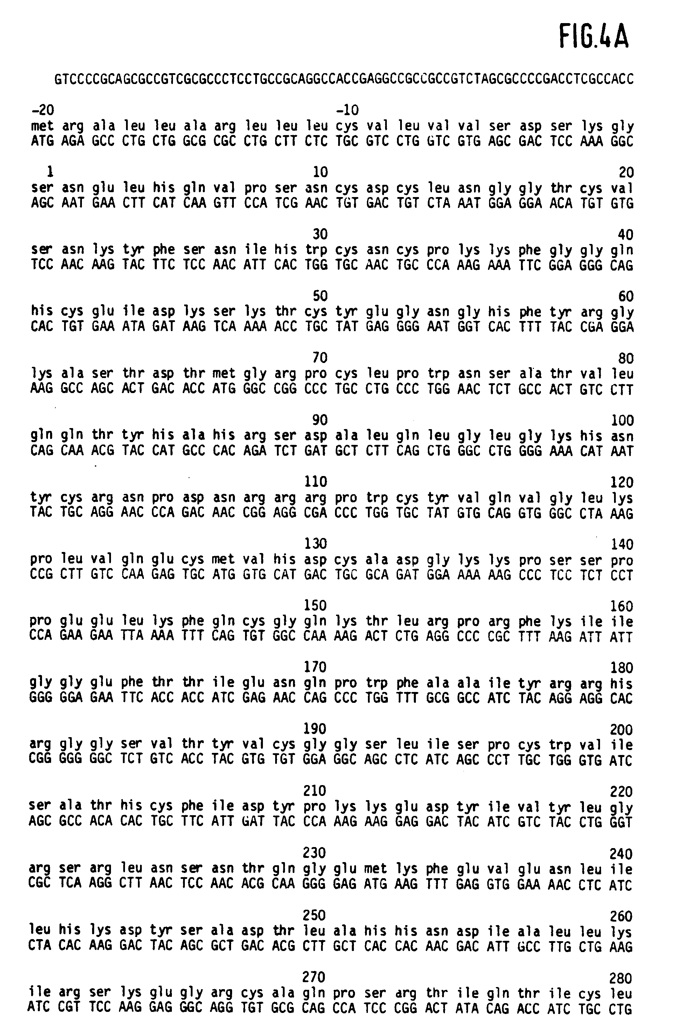

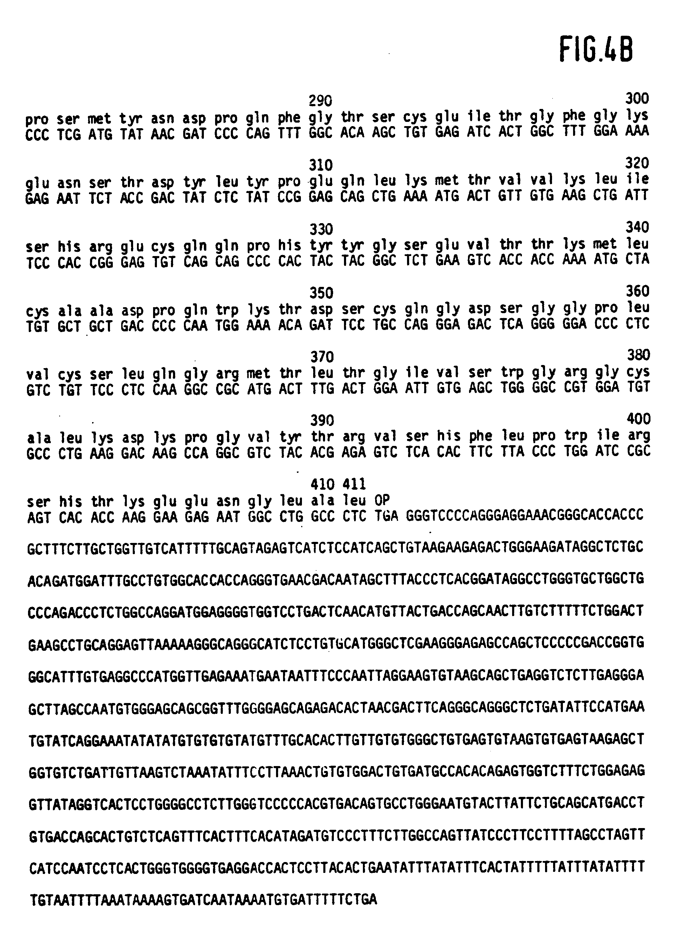

The present invention provides an unglycosylated polypeptide having an intact lysine-isoleucine bond at the position corresponding to amino acids 158-159 in the native prourokinase molecule, and having sufficient of the amino acid sequence set out in Figures 4A and 4B such that, on activation, the activated polypeptide exhibits the enzymatic activity of human urokinase. The polypeptide of the invention can be, for example, an unglycosylated prourokinase protein or Met-prourokinase protein. It is preferred that the polypeptide has the sequence Lys-Ile-Ile-Gly-Gly, at positions corresponding to amino acids 158-162 in the native prourokinase molecule and, more particularly, that it has the amino acid sequence or substantially the amino acid sequence as depicted in Figures 4A and 4B.

The present invention includes an isolated DNA sequence encoding the polypeptide of the invention, which sequence will normally be linked with a second DNA sequence capable of effecting expression of the first, and is further directed to replicable DNA expression vehicles harboring gene sequences encoding the polypeptide of the invention in expressible form. Further, the present invention is directed to a microorganism, e.g. E. coli, transformed with the expression vehicles described above and to microbial cultures thereof capable of directing production of the polypeptide of the invention.

In still further aspects, the invention includes a composition comprising a therapeutically effective amount of the polypeptide of the invention together with a pharmaceutically acceptable carrier and further includes the polypeptide of the invention for use in a method of treatment of the human or animal body for the treatment of vascular diseases. Still further, this invention provides a process for producing a polypeptide by expressing a DNA sequence of the invention to form an unglycosylated polypeptide that, on activation, e.g. by proteolytic cleavage, is converted to a polypeptide that exhibits the enzymatic activity of human urokinase.

Reference herein to the expression "human urokinase polypeptide" connotes polypeptide in bioactive form, produced by microbial culture or optional in vitro processing and comprising the enzymatic portion corresponding to native material. Human urokinase polypeptide, according to the present invention, is thus provided 1) in full length, in contradistinction to material hitherto isolated from natural sources or 2) in other, bioactive forms bearing the sites of the enzymatic portion found essential for plasminogen activation or 3) having a methionine first amino acid or a signal polypeptide or conjugated polypeptide other than the signal polypeptide fused at the N-terminus of the enzymatic portion, the methionine, signal or conjugate polypeptide being specifically cleavable in an intra- or extracellular environment (see reference 12). In any event, the thus produced human urokinase polypeptides are recovered and purified to levels fitting them for use in the treatment of various cardiovascular conditions or diseases.

### Description of Preferred Embodiments

### A. Microorganism Cultures

### 1. Bacterial Strains/Promoters

The work described herein was performed employing, inter alia, the microorganism E. coli K-12 strain GM 48 (thr⁻, leu⁻, B₁⁻, lacY1, gal K12, gal T22, ara 14, ton A31, tsx 78, Sup E44, dam 3, dcm 6) deposited with the American Type Culture Collection on April 9, 1982 (ATTC No. 39099) and E. coli K-12 strain 294 (end A, thi⁻, hsr⁻, ₖhsm⁺), described in reference (16), deposited with the American Type Culture Collection, ATCC Accession No. 31446, on October 28, 1978. However, various other microbial strains are useful, including known E. coli strains such as E. coli B, E. coli X 1776 (ATCC No. 31537, deposited July 3, 1979) and E. coli W 3110 (F⁻, λ⁻, protrophic) (ATCC No. 27325 deposited February 10, 1972), or other microbial strains many of which are deposited and (potentially) available from recognized microorganism depository institutions, such as the American Type Culture Collection (ATCC)--cf. the ATCC catalogue listing. See (17). These other microorganisms include, for example, Bacilli such as Bacillus subtilis and other enterobacteriaceae among which can be mentioned as examples Salmonella typhimurium, Serratia marcesans, and Pseudomonas. utilizing plasmids that can replicate and express heterologous gene sequences therein.

As examples, the beta lactamase and lactose promoter systems have been advantageously used to initiate and sustain microbial production of heterologous polypeptides. Details relating to the make-up and construction of these promoter systems can be had by reference to (18) and (19). More recently, a system based upon the tryptophan pathway, the so-called trp promoter system, has been developed. Details relating to the make-up and construction of this system can be had by reference to (20) and (21). Numerous other microbial promoters have been discovered and utilized and details concerning their nucleotide sequences enabling a skilled worker to ligate them functionally within plasmid vectors, have been published. -- See (22).

### B. Vector Systems

### 1. Expression in Bacterial Systems

Expression plasmids for bacterial use, e.g., E. coli are commonly derived using BR322 (37) as vector and appropriately inserting the heterologous gene sequence together with translational start and stop signals in operable reading phase with a functional promoter, taking advantage of common or synthetically created restriction sites. The vector will carry one or more phenotypic selection characteristic genes and an origin of replication to insure amplification within the host. Again, the heterologous insert can be aligned so as to be expressed together with a fused presequence, derivable for example from the trp system genes.

### Brief Description of the Drawings

Figure A is a schematic diagram of urokinase polypeptides. Low molecular weight urokinase begins at amino acid 136 and ends at amino acid 411. High molecular weight urokinase starts at amino acid 1 and terminates at amino acid 411. Conversion of the one chain form of both high and low molecular weight urokinase to the bioactive two-chain form occurs by proteolytic cleavage between amino acid 158 and 159. Pre-urokinase begins at amino acid-20. The depicted conformational positioning of the disulfide bonds is based on analogy with other serine proteases.

Figures 1A to 1C depict the nucleotide sequence and restriction endonuclease map of the plasmid pD2 cDNA insert for low molecular weight 33000 dalton urokinase bioactive polypeptide. The nucleotide portion of the synthetic deoxyoligonucleotide CB6B probe is underlined.

Figures 2A,B depict the deduced amino acid sequence of the cDNA sequence of figure 1, the amino acids of the cDNA insert portion being numbered 1 to 279.

Figures 3A to 3C depict the nucleotide sequence and restriction endonuclease map of the cDNA for full length human urokinase polypeptide The CB6B probe is likewise underlined.

Figures 4A,B depict the deduced amino acid sequence for full length urokinase polypeptide from the cDNA sequence of Figure 3.

Figure 5 illustrates the construction of plasmid pUK33trpLE_{L} for expression of long fusion -33000 dalton protein.

Figure 6 illustrates the construction of plasmid pUK33trpLE_{S} for expression of short fusion -33000 dalton protein.

Figure 7 shows the construction of the plasmid for direct expression of the 33000 dalton polypeptide.

Figure 8 illustrates another construction of a plasmid for direct expression of the 33K dalton polypeptide.

Figures 9 and 10 illustrate the construction of plasmids for the direct expression of 54K urokinase polypeptide and a precursor form of 54K urokinase polypeptide.

Figure 11 illustrates the time dependent activation of, and the requirement for, plasminogen in a plasmin assay by urokinase polypeptide produced as described herein.

Figure 12 illustrates the plasminogen activating activity of the urokinase polypeptide extracts hereof and the inhibition of that activity by antibodies raised against natural urokinase.

### Detailed Description

### A. Source of Urokinase mRNA

Detroit 562 (human pharangeal carcinoma) cells (38)(ATCC No. CCL 138) were cultured to confluency in Eagle's minimal essential medium (39) supplemented to contain 3 percent sodium bicarbonate (pH 7.5), 1 percent L-glutamine (Irvine), 10 percent fetal bovine serum, 1 percent sodium pyruvate (Irvine), 1 percent non-essential amino acids (Irvine), 2.4 percent HEPES (pH 7.5), 50 µg/ml Garamycin, and incubated at 37°C in a 5 percent CO₂ atmosphere. Confluent cells were harvested by centrifugation after treatment with 0.25 percent trypsin for 15 minutes at 37°C.

### B. Messenger RNA Isolation

Total RNA from Detroit 562 cells was extracted essentially as reported by Lynch et al. (40). Cells were pelleted by centrifugation and approximately one gram of cells was then resuspended in 10 ml of 10 mM NaCl, 10 mM Tris·HCl (pH 7.4), 1.5 mM MgCl₂. Cells were lysed by the addition of non-ionic detergent NP-40 to a final concentration of 1 percent. Nuclei were removed by centrifugation and the RNA was further purified by two successive phenol (redistilled)/chloroform: isoamyl alcohol extractions at 4°C. The aqueous phase was made 0.2 M NaCl and total RNA was precipitated by addition of two volumes of 100 percent ethanol and overnight storage at -20°C. Following centrifugation polyA mRNA was purified from total RNA by oligo-dT cellulose chromatography (41). Yields from 1 gram of cells were typically 10-15 mg of total RNA and ∼2 percent of that was polyA mRNA.

### C. Size Fractionation of polyA mRNA

Size fractionation of 200 µg polyA mRNA was achieved by electrophoresis through an acid-urea gel composed of 1.75 percent agarose, 25 mM sodium citrate (pH 3.8) and 6 M urea (40,42). Electrophoresis was performed for 7 hours at 25 mA and 4°C. The gel was then fractionated manually with a razor blade. Individual slices of the gel were melted at 70°C, diluted into 12 mls 10 mM NaCl, 10 mM Tris·HCl (pH 7.4), 1.5 mM MgCl₂, 0.1 percent SDS and extracted twice with water saturated, redistilled phenol and once with chloroform. Fractions were then ethanol precipitated and subsequently translated in vitro (43) in order to determine the affected size fractionation and integrity of the polyA mRNA.

### D. Preparation of Oligo-dT Primed Colony Library Containing Urokinase DNA Sequences

Poly A mRNA was size-fractionated on acid urea gels. mRNA fractions greater than 12S were pooled and used as template for oligo-dT primed preparation of double stranded cDNA by standard procedures (44,45). The cDNA was size fractionated on 6 percent polyacrylamide gel electrophoresis and 132 ng of ds cDNA greater than 350 basepairs in length was obtained by electroelution. A 30 ng portion of ds cDNA was extended with deoxy C residues using terminal deoxynucleotidyl transferase (46) and annealed with 200 ng of the plasmid pBR322 (37) which had been similarly tailed with deoxy G residues at PstI site (46). Each annealed mixture was then transformed into E. coli K12 strain 294 (ATCC No. 31446). Approximately 10000 ampicillin sensitive, tetracycline resistant transformants were obtained.

### E. Preparation of Synthetic DNA Oligomers for Use as Urokinase Screening Probes

Eight synthetic DNA oligomers 14 bases long were designed complementary to mRNA based on the Met-Tyr-Asn-Asp-Pro amino acid sequence of a urokinase cyanogen bromide polypeptide fragment designated CB6. These eight deoxyoligonucleotides were synthesized by the phosphotriester method (47) in the following pools of two: (CB6A) 5' GGGTCGTTA/GTACAT 3', (CB6B) 5' GGATCGTTA/GTACAT 3', (CB6C) 5' GGGTCATTA/GTACAT 3', (CB6D) 5' GGATCATTA/GTACAT 3'. Each pool of two oligomers was then radioactively phosphorylated as follows: 250 ng of deoxyoligonucleotide were combined in 25 µl of 60 mM Tris·HCl (pH 8), 10 mM MgCl₂, 15 mM beta-mercaptoethanol, and 100 µCi (γ-³²P) ATP (Amersham, 5000 Ci/mMole). 5 units of T4 polynucleotide kinase were added and the reaction was allowed to proceed at 37°C for 30 minutes and terminated by addition of EDTA to 20 mM.

### F. Screening of Oligo dT Primed Colony Library for Urokinase Sequences

∼10000 colonies were individually inoculated into wells of microtitre dishes containing LB (48) + 5 µg/ml tetracycline and stored at -20°C after addition of DMSO to 7 percent. Individual colonies from this library were transferred to Schleicher and Schuell BA85/20 nitrocellulose filters and grown on agar plates containing LB + 5 µg/ml tetracycline. After ∼10 hours growth at 37°C the colony filters were transferred to agar plates containing LB + 5 µg/ml tetracycline and 12.5 µg/ml chloramphenicol and reincubated overnight at 37°C. The DNA from each colony was then denatured and fixed to the filter by a modification of the Grunstein-Hogness procedure (49). Each filter was floated for 3 minutes on 0.5 N NaOH, 1.5 M NaCl to lyse the colonies and denature the DNA then neutralized by floating for 15' on 3 M NaCl, 0.5 M Tris·HCl (pH 7.5). The filters were then floated for an additional 15 minutes on 2XSSC, and subsequently baked for 2 hours in an 80°C vacuum oven. The filters were prehybridized for ∼2 hours at room temperature in 0.9 M NaCl, 1X Denhardts, 100 mM Tris·HCl (pH 7.5), 5 mM Na-EDTA, 1 mM ATP, 1 M sodium phosphate (dibasic), 1 mM sodium pyrophosphate, 0.5 percent NP-40, and 200 µg/ml E. coli t-RNA, and hybridized in the same solution overnight essentially as described by Wallace et al. (50) using ∼40x10⁶ cpm of each kinased CB6 deoxyoligonucleotide pool of 2. After extensive washing at 37°C in 6X SSC, 0.1 percent SDS, the filters were exposed to Kodak XR-5 X-ray film with DuPont Lightning-Plus intensifying screens for 16-24 hours at -80°C. Two colonies indicated hybridization with the mixture of eight probes: UK513dT69D2 (pD2) and UK513dT73D12 (pD12).

### G. Characterization of pD2 and pD12 Plasmid DNA

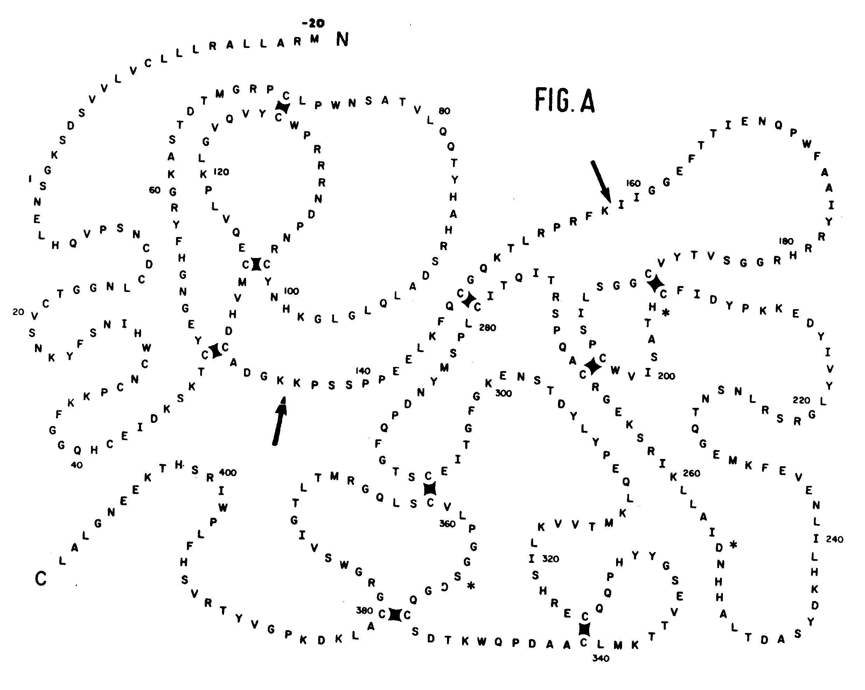

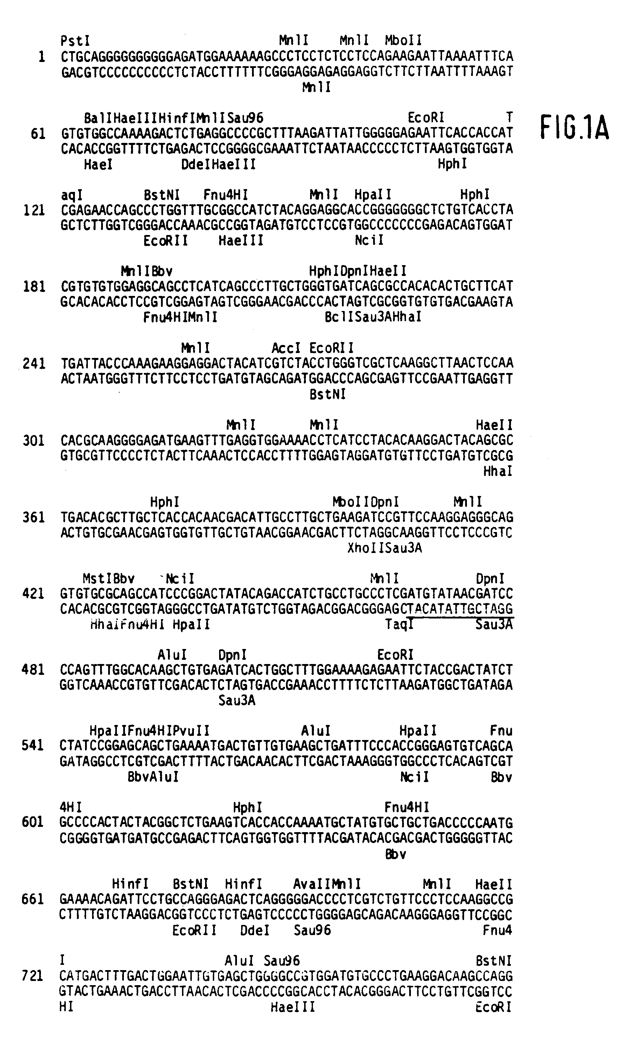

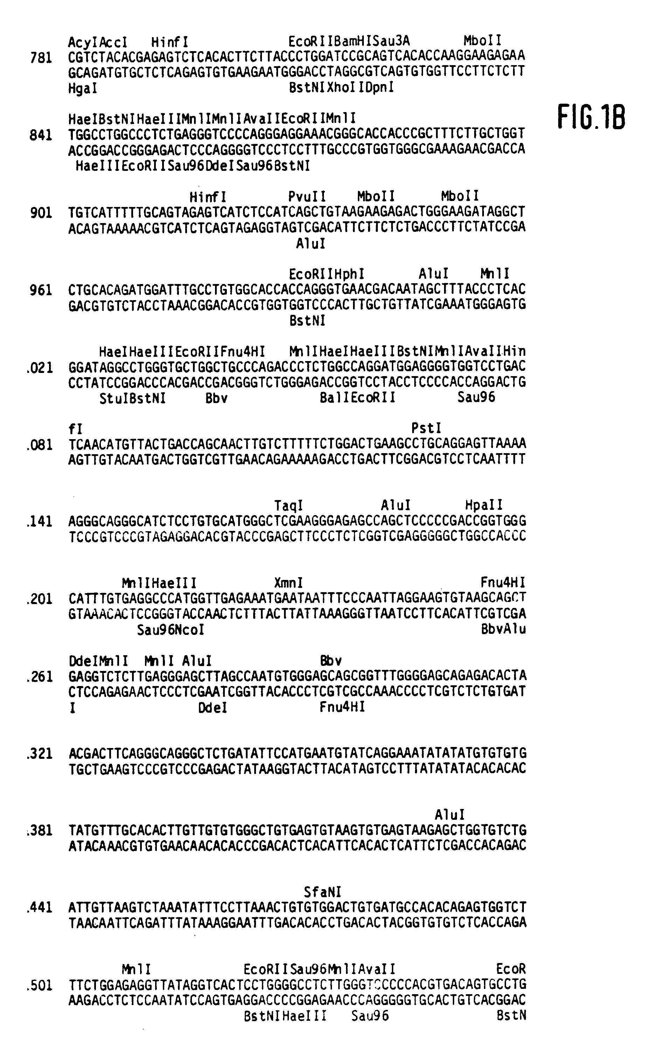

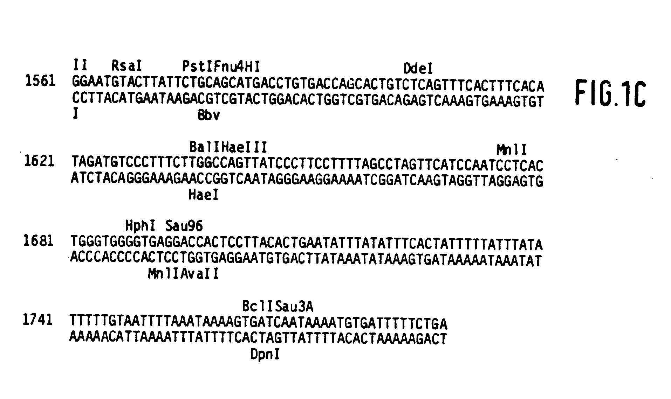

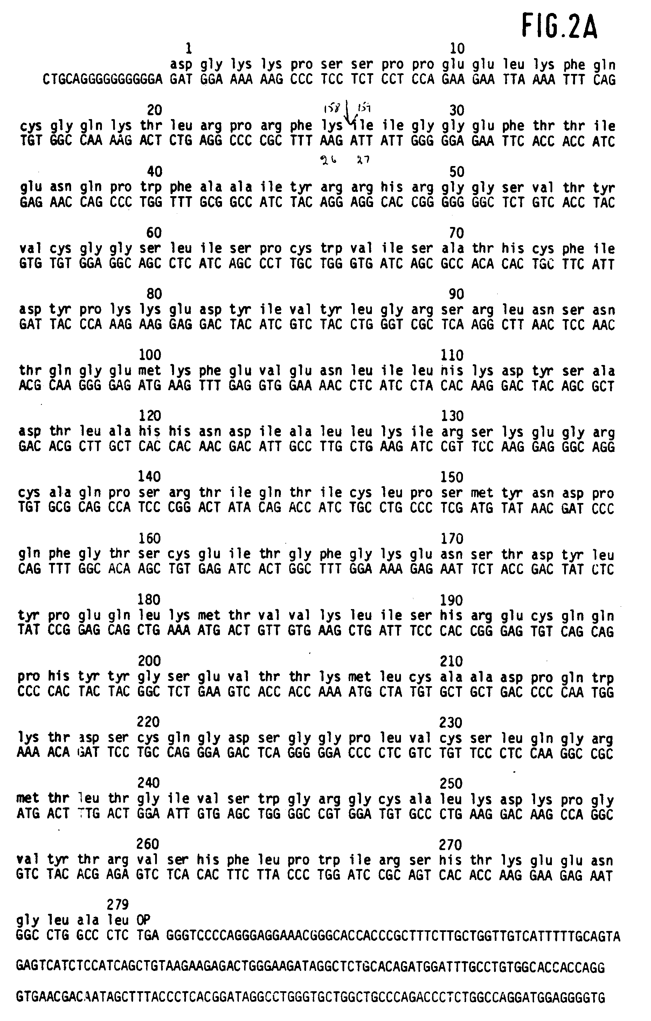

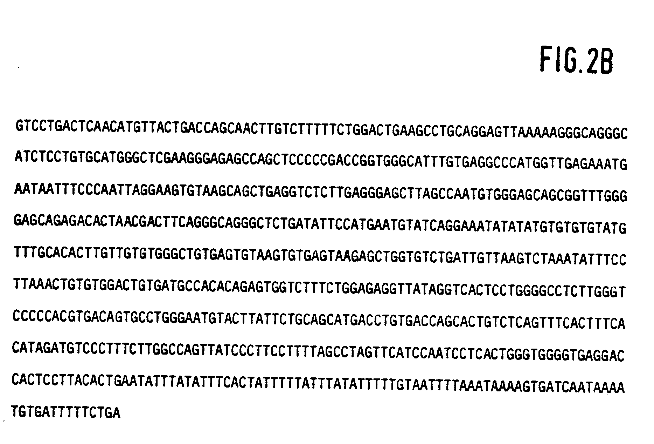

Plasmid DNA isolated from E. coli colony UK513dT69D2 and UK513dT73D12 by a rapid mini screen method (51) was subjected to PstI restriction endonuclease analysis. This analysis strongly suggested that pD2 and pD12 are identical. Each plasmid DNA has 3 PstI restriction fragments that comigrate when electrophoresed through a 6 percent polyacrylamide gel. The complete nucleotide sequence of the plasmid pD2 cDNA insert was determined by the dideoxynucleotide chain termination method (52) after subcloning the PstI restriction fragments into the M13 vector mp7 (53). Figure 1 presents the nucleotide sequence and Figure 2 presents the translated nucleotide sequence of the cDNA insert fragment of pD2. The entire coding region of low molecular weight (33K) urokinase was isolated on this one large fragment of pD2. The nucleotide sequence of the CB6B (5' GGATCGTTA/GTACAT) deoxyoligonucleotide includes nucleotides 466 through 479 according to this map. A typical serine protease active site (gly asp ser gly gly pro) is present between amino acids 222 and 227. The coding region consists of 838 basepairs or 279 amino acids of the carboxy terminal portion of high molecular weight (54K) urokinase. The stop codon UGA at amino acid position 280 begins ca. 935 nucleotides of 3' untranslated sequence up to the poly A sequence. Because only 31413 daltons of full length urokinase were encoded by the cDNA insert of pD2 it was necessary to construct additional colony banks containing urokinase sequences in order to identify high molecular weight urokinase polypeptide.

### H. Construction of Two Different Specifically Primed Colony Banks for Amino Terminal Extension of the Existing Urokinase Clone

The first specifically primed cDNA bank utilized a 45 basepair urokinase DNA restriction endonuclease fragment beginning with HaeII in position 225 and ending with AccI in position 270 (Figure 1) as a primer rather than oligo dT₁₂₋₁₈. This fragment was heat denatured in the presence of 20 µg unfractionated Detroit 562 polyA mRNA and cDNA was prepared according to procedures referenced in Section D. 11.5 ng of double-stranded cDNA greater than 200 bp were electroeluted from a 6 percent polyacrylamide gel, and used to generate approximately 6000 clones in E. coli 294.

A second specifically primed cDNA bank called UK89CB6 of about 4000 colonies was constructed using a pool of 4 µg polyA mRNA acid-urea agarose gel fraction 8 and 4 µg polyA mRNA from fraction 9 (Section C). 250 ng of each CB6 deoxyoligo- nucleotide pool (Section E) were used as primer rather than oligo dT₁₂₋₁₈.

### I. Screening of Full Length Colony Bank

Full length cDNA containing colonies were transferred directly to nitrocellulose filters then grown at 37°C. The colonies were lysed and the DNA was denatured and fixed to the filters as described in Section F (49). A ³²P-labelled DNA probe was prepared (54) from a 143 basepair HinfI to HaeII restriction endonuclease fragment from the cDNA insert of pD2 and hybridized (55) with the filter fixed full length cDNA. 8x10⁶ CPM of the probe was hybridized for 16 hours then washed as described (55) and exposed to X-ray film. Two colonies demonstrated strong hybridization: A3 and E9.

### J. Characterization of Full Length Urokinase cDNA's pA3 and pE9

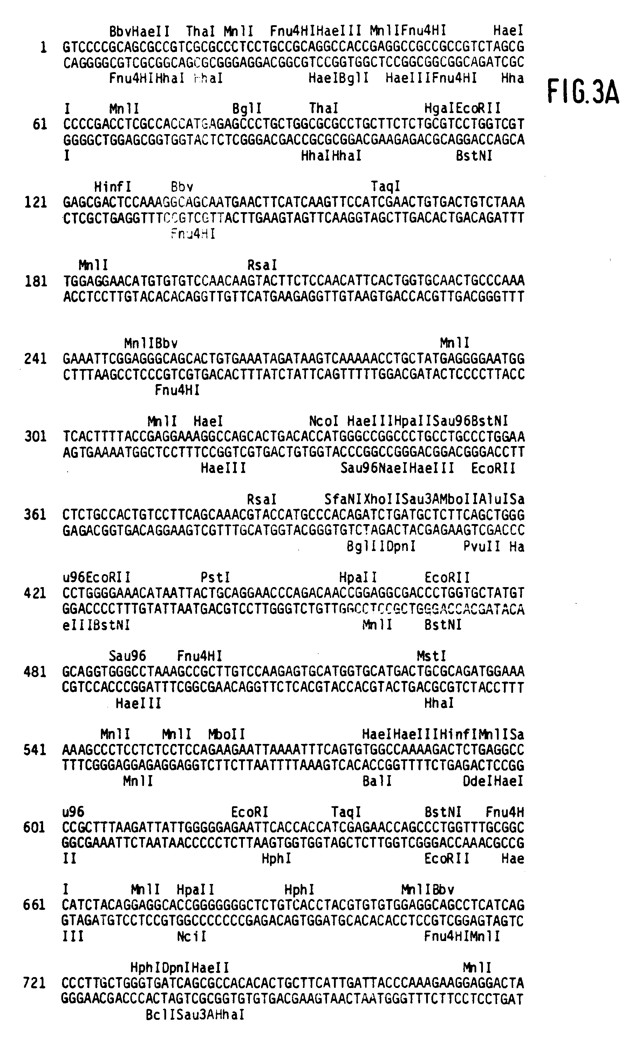

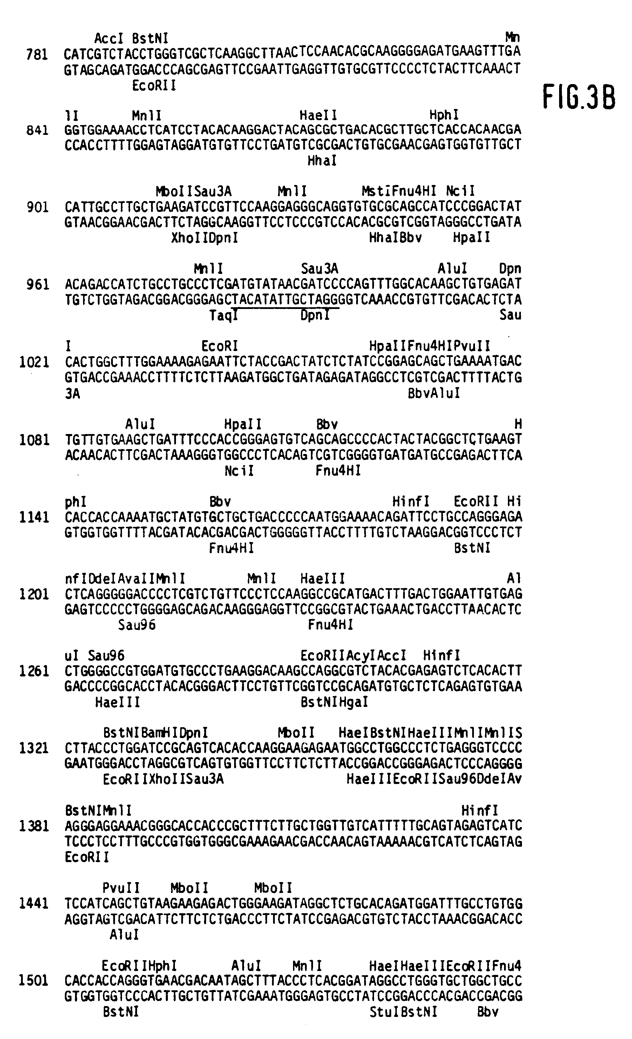

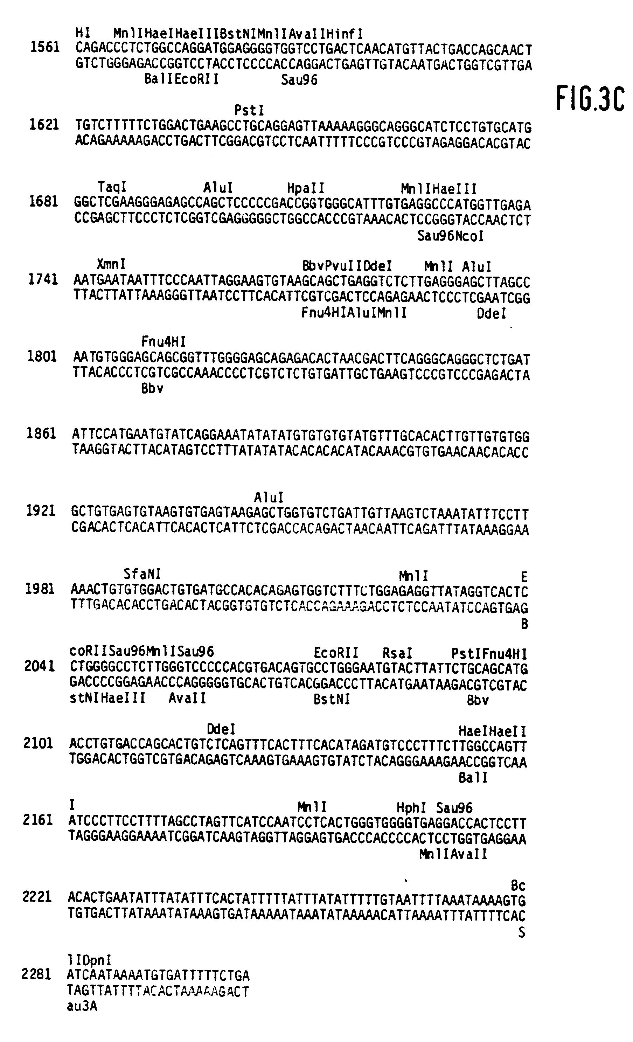

PstI restriction analysis of A3 plasmid DNA showed cDNA insert fragments of ∼360 bp and ∼50 bp, and of E9 plasmid DNA, one fragment at ∼340 bp. PstI EcoRI double restriction of each plasmid DNA revealed a common cDNA insert fragment of ∼190 bp as predicted where each plasmid DNA encoded urokinase sequence information 5' to the HaeII AccI primer fragment. Plasmid pA3 has an additional PstI EcoRI cDNA insert fragment of 185 bp and E9 a 160 bp additional fragment. The larger ∼360 bp PstI cDNA insert fragment of pA3 was subcloned into the M13 vector mp7 (53) and sequenced by the dideoxynucleotide chain termination method (52). The urokinase coding sequence of pA3 is located from approximately position 405 to position 785 in the cDNA sequence for full length urokinase protine in Figure 3.

### K. Screening of Urokinase Colony Bank UK89CB6

DNA from ∼1900 UK89CB6 cDNA insert containing colonies was denatured and fixed to nitrocellulose filters as previously described in Section F. A ³²P-labelled DNA probe was prepared (54) from the 146 bp PstI HinfI fragment of the cDNA insert fragment of pA3. 40x10⁶ CPm of this probe was then hybridized to the filter bound DNA of UK89CB6 colonies for 16 hours then washed as described (55) and exposed to X-ray film. Two colonies demonstrating positive hybridization were UK89CB6F1 (pF1) and UK89CB6H10 (pH10).

### L. Characterization of pFI and pH10 Urokinase cDNAs

PstI restriction of pF1 demonstrates cDNA insert fragments of ∼450 bp and ∼125 bp, and pH10 shows one PstI cDNA insert fragment of ∼500 bp. PstI EcoRI double restriction of pF1 indicates no EcoRI restriction site and has cDNA insert fragments identical to those of the PstI restriction alone. pH10 does demonstrate an EcoRI restriction site, producing cDNA insert fragments of ∼375 bp and ∼110 bp. This EcoRI site of pH10 is probably the same EcoRI site as noted at position 627 (Figure 3). The pF1 cDNA insert does not contain this EcoRI restriction site.

pF1, having a cDNA insert fragment longer than that of pH10, was selected for sequencing. Both PstI restriction fragments of the pF1 cDNA insert were sequenced by M13 subcloning and dideoxy sequencing. The composite nucleotide sequence of UK cDNA inserts from pF1, pA3 and pD2 encoding the entire amino acid sequence of high molecular weight full length urokinase is shown in Figure 3. The urokinase coding sequence of pF1 is depicted from position 1 approximately to position 570. The urokinase coding sequence of pD2 is located from position 532 to position 2304 in Figure 3.

The amino terminal serine at amino acid position one as determined by amino acid sequence analysis is shown in Figures A and 4. The preceding 20 amino acids at the amino terminus beginning with met and ending with gly probably serves as a signal sequence for the secretion of the remaining 411 amino acids of high molecular weight urokinase. This putative signal sequence has features, such as size and hydrophobicity (56,57), in common with other characterized signal sequences.

Trypsin cleavage sites rendering 33K two-chain low molecular weight urokinase from high molecular weight urokinase are as follows: lys at position 136 is the amino terminal amino acid of the short chain and ile at position 159 is the amino terminus of the long chain (Figures A, 4).

### M. Expression of Low Molecular Weight Derivatives of Urokinase polypeptide in E. coli

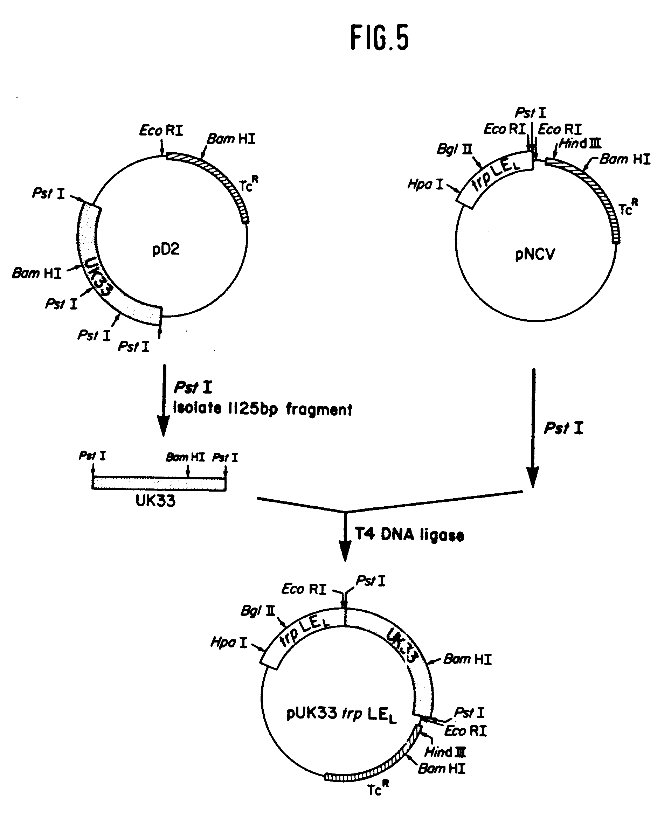

### 1. Long trp LE fusion (Figure 5)

A plasmid (pNCV, 58) was constructed which has the following properties: 1) the plasmid is a derivative of pBR322 and is present in about 20 copies per cell. 2) the plasmid makes its E. coli host resistant to tetracycline. 3) the plasmid contains an inducible tryptophan promoter, which directs the synthesis of a protein consisting of a fusion between the trp leader peptide and the trp E structural gene (LE fusion gene). 4) A unique PstI restriction site was constructed in the trp E gene, which can be used to clone PstI DNA fragments, by converting the EcoRI site at the distal end of the LE gene in plasmid pSOM7Δ1Δ4 to a PstI site flanked by two EcoRI sites using a synthetic sequence: 
AATTCTGCAG
    GACGTCTTAA.
The DNA fragment containing the trp promoter and LE gene was then introduced into plasmid pBR322 to give plasmid pNCV (47A).

The urokinase PstI fragment from nucleotide position 5 (the Pst I 5'- cleavage site) to nucleotide position 1130 (Figure 1) was cloned into the PstI site of pNCV in such a way that a fusion protein is made upon induction of the trp promoter. The N-terminal part is trp LE and the C-terminal part is low molecular weight urokinase polypeptide.

With reference to Figure 5, 5 µg of plasmid pUK513dT69D2 (pD2) was digested with 20 units PstI and the 1125 bp cDNA insert fragment encoding low molecular weight urokinase was isolated by 6 percent polyacrylamide gel electrophoresis. ∼1 µg of this insert was electroeluted from the gel, phenol/chloroform extracted and ethanol precipitated. 1 µg of the vector plasmid pNCV (58) gas digested with 10 units PstI and ethanol precipitated after phenol/chloroform extraction. ∼100 ng of this 1125 bp fragment was combined with ∼100 ng PstI digested pNCV in 20 µl of 20 mM Tris·HCl (pH 7.5), 10 mM MgCl₂, 10 mM DTT, 2.5 mM ATP and 30 units of T4 DNA ligase. After overnight ligation at 14°C, one half of the mixture was transformed in E. coli K12 strain 294. BamHI digestion of the DNA from twelve transformants showed 3 with the proper orientation. Expression of this plasmid (pUK33trpLE_{L}) (Figure 5) in E. coli yielded a long trp LE fusion protein including 33000 urokinase polypeptide. The 33000 urokinase polypeptide is activated by cleavage a trypsin-like active enzyme between positions 3 and 4 and positions 26 and 27 (see Figure 2).

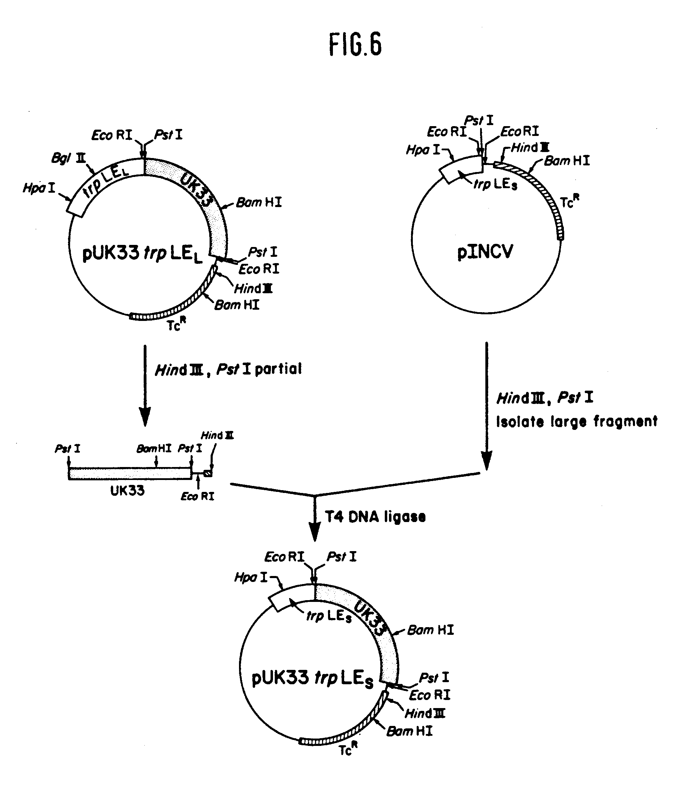

### 2. Short trp LE fusion (Figure 6)

Plasmid pINCV was constructed. It is similar to pNCV (see supra.) in every respect except that the majority of the trp E gene is deleted. In this plasmid the BglII site was converted to a PstI site and the region between this new PstI site and the original PstI site was deleted. ∼100 ng of pINCV was digested with PstI and HindIII then phenol/CHCl₃ extracted and ethanol precipitated. ∼3 µg of pUK33trpLE_{L} (Figure 6) was digested to completion with HindIII and partially digested with PstI to yield a PstI HindIII DNA fragment of 1150 bp which was purified by electroelution after electrophoresis on a 6 percent polyacrylamide gel. The PstI site within the structural UK gene was spared from digestion. All of the PstI HindIII digested pINCV was mixed with ∼50 ng of the 1150 bp HindIII, partial PstI fragment of pUK33trpLE_{L} and ligated overnight at 14°C. This mixture was then transformed into E. coli K12 strain 294. BamHI digestion confirmed the proper construction of this plasmid (pUK33trpLE_{S}) (Figure 6). Expression of this plasmid in E. coli yielded a fusion protein from which 33,000 urokinase polypeptide is activated, as described supra.

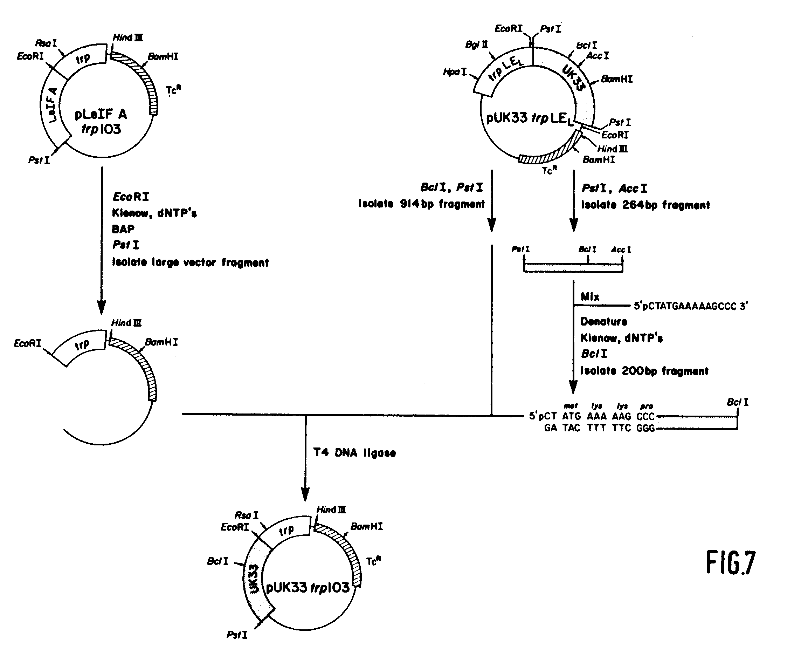

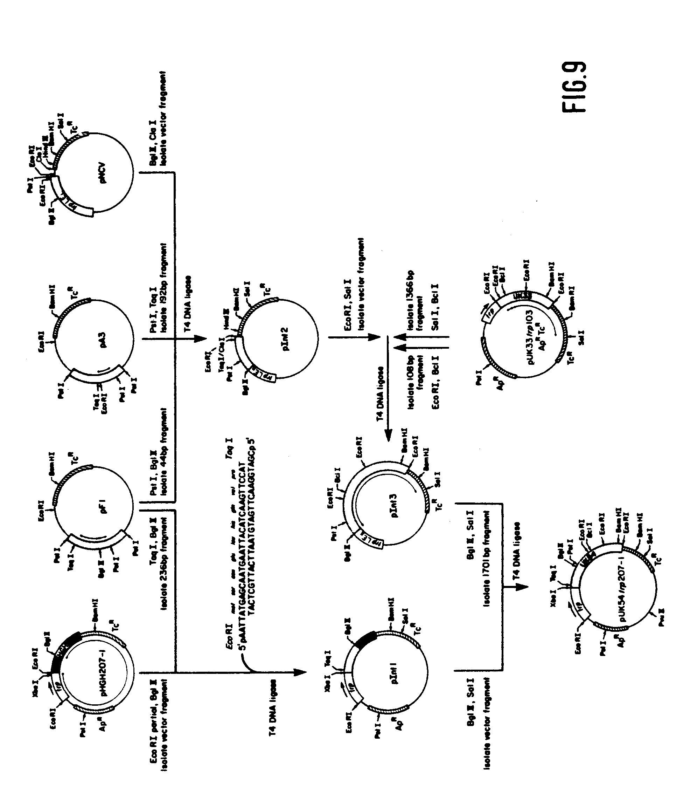

### 3. Direct Expression of 33K Urokinase polypeptide (Figures 7 and 9)

A urokinase DNA fragment beginning with nucleotide 16 (Figure 1), was cloned into a pBR322 derivative resulting in a construction in which the trp promoter is positioned directly in front of this urokinase fragment encoding low molecular weight urokinase polypeptide. The plasmid pLeIFAtrp103 (Figure 7) is a derivative of the plasmid pLeIFA25 (58) in which the Eco RI site distal to the LeIFA gene has been removed (59). 10 µg of pLeIFAtrp103 (Figure 7) was digested with 20 units EcoRI phenol/CHCl₃ extracted and ethanol precipitated. The EcoRI cohesive ends of the plasmid DNA molecules were extended to flush ends using 12 units of DNA Polymerase I in a 50 µl reaction containing 60 mM NaCl, 7 mM MgCl₂, 7 mM Tris·HCl (pH 7.4) and 1 mM in each ribonucleotide triphosphate. The reaction was incubated at 37°C for 1 hour, extracted with phenol/CHCl₃ and precipitated with ethanol. The DNA was then resuspended in 50 µl of 10 mM Tris·HCl (pH 8), 1 mM EDTA and treated with 500 units Bacterial Alkaline Phosphatase for 30 minutes at 65°C, twice extracted and ethanol precipitated. After digestion with PstI the mixture was electrophoresed on a 6 percent polyacrylamide gel and the ∼3900 bp vector fragment was electroeluted.

The plasmid PUK33trpLE_{L} was transformed into E. coli K12 strain GM48 (deoxyadenosine methylase⁻) in order that DNA purified from this E. coli strain could be digested with restriction endonuclease BclI (60). 4 µg of this DNA were treated for 1 hour at 50°C with 6 units of BclI (in 75 mM KCl, 6 mM Tris·HCl (pH 7.4), 10 mM MgCl₂, 1 mM DTT), then made 50 mM NaCl and digested with 10 units PstI. 6 percent gel electrophoresis was performed and the 914 bp fragment was electroeluted.

A 14 nucleotide DNA primer encoding the amino acid sequence met lys lys pro was synthesized by the phosphotriester method (47) and has the following sequence: 
MetLysLysPro
5' CTATGAAAAAGCCC 3'
500 ng of this primer were phosphorylated at the 5' end with 10 units T4 DNA Kinase in a 20 µl reaction containing 0.5 mM ATP. The 264 bp PstI AccI cDNA insert fragment of pUK33trpLE_{L} (grown in E. coli GM48) was isolated. ∼500 ng of this fragment resuspended in 10 µl of deionized water were mixed with the 20 µl of the phosphorylated primer, heated to 95°C for 3 minutes and quick frozen in a dry-ice ethanol bath. The denatured DNA solution was made 60 mM NaCl, 7 mM MgCl₂, 7 mM Tris·HCl (pH 7.4), 1 mM in each deoxy ribonucleotide triphosphate and 12 units DNA polymerase I large fragment was added. After 2 hours incubation at 37°C this primer repair reaction was phenol/CHCl₃ extracted and ethanol precipitated and digested to completion with BclI at 50°C. The reaction mixture was then run on a 6 percent polyacrylamide gel and ∼50 ng of the 200 bp amino-terminal blunt-end to BclI fragment was electroeluted. Subsequently, ∼50 ng of the blunt-BclI primer-repair fragment, ∼100 ng of the BclI PstI carboxy-terminal fragment and ∼100 ng of the ∼3900 bp vector fragment were ligated overnight at 14°C and transformed into E. coli 294. EcoRI digestion of a number of transformants indicated the proper construction and DNA sequence analysis proved the desired sequence through the initiation codon of this new plasmid, pUK33trp103 (Figure 7). In this construction, the N-terminal methionine is followed by two lysines which in turn are followed by the amino acid sequence 5 through 279 as depicted in Figure 2A. Expression of this plasmid in E. coli resulted in the synthesis of low molecular weight urokinase polypeptide. This polypeptide was activated with a trypsin-like active enzyme as described supra which serves to cleave the N-terminal lysine pair and cleaves between lysine in position 26 and isoleucine in position 27 (Figure 2A).

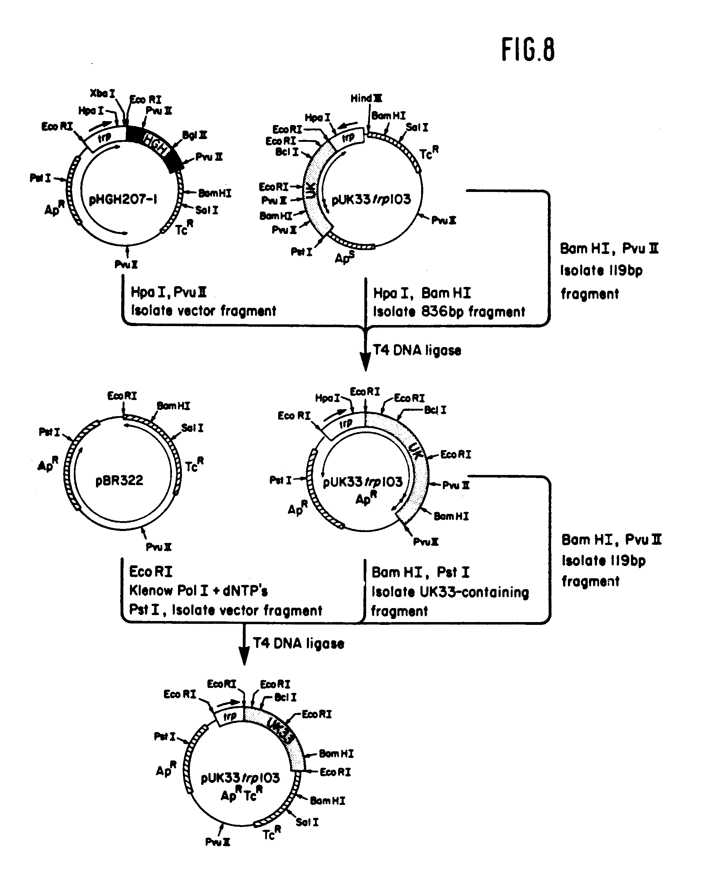

We found it desirable to construct a derivative plasmid of pUK33trp103 that would confer tetracycline resistance to its host cell. Figure 8 depicts the following construction of pUK33trp103Ap^{R-}Tc^{R}. 5µg of pHGH207-1 (See infra) was digested with HpaI and PvuII. The vector fragment was isolated and purified. 5µg of pUK33trp103 was digested with HpaI and BamH1, electrophoresed on 6 percent polyacrylamide and the 836 bp DNA fragment was purified. A second 5µg aliquot of pUK33trp103 was digested with BamH1 and PvuII for isolation and purification of the 119 bp DNA fragment. Equal molar amounts of each of these three DNA fragments were ligated overnight at 14°C and used to transform E. coli 294. Restriction endonuclease analysis of plasmid DNA from several ampicillin resistant transformants verified the proper construction of pUK33trp103Ap^{R} and the reversal in orientation of the trp promoter/UK33 encoding DNA.

∼5µg pBR322 DNA was digested with EcoR1 and the cohesive ends were filled in with Klenow Pol1. After Pst1 digestion, the large vector fragment containing the DNA that encodes tetracycline resistance, the origin of replication, and a portion of the ampicillln resistance gene was isolated and purified. ∼5µg of pUK33trp103 Ap^{R} was digested with BamH1 and Pst1. The DNA fragment encoding the remaining portion of the ampicillin resistance gene, the trp promoter and most of low molecular weight urokinase was purified. Approximately equal molar amounts of these two DNA fragments and the 119 bp BamH1-PvuII DNA fragment from pUK33trp103Ap^{R} was ligated overnight at 14°C to complete the construction of pUK33trp103Ap^{R}Tc^{R}. This plasmid was employed in the construction of a plasmid designed to express high molecular weight full length urokinase polypeptide (see Section N and Figure 9).

### N. Expression of High Molecular Weight Derivatives of Urokinase polypeptide

### 1. Direct Expression of 54K Urokinase polypeptide

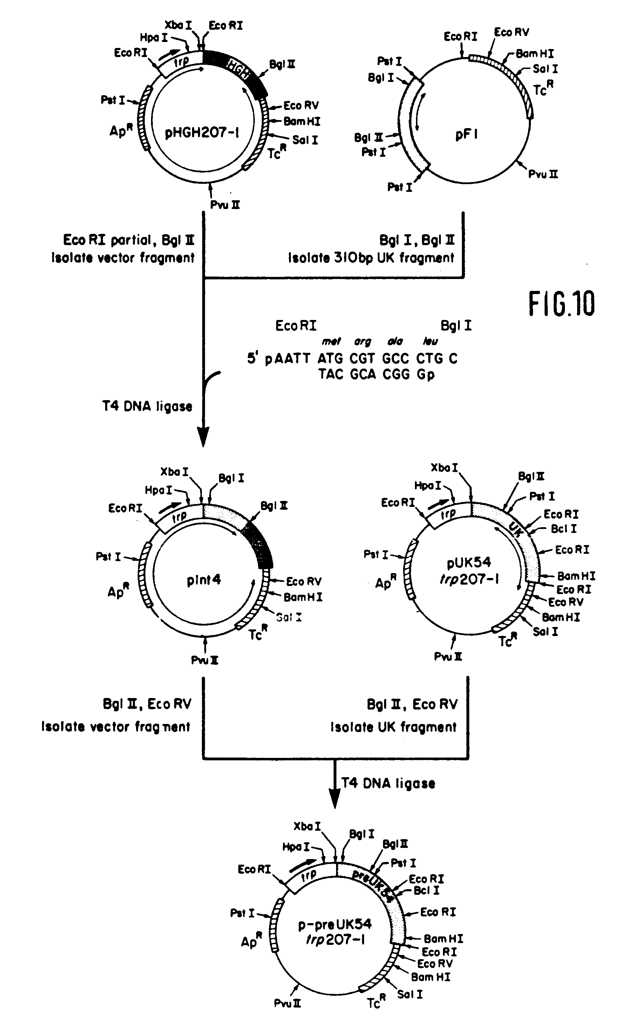

Figure 10 illustrates construct for full length urokinase polypeptide. Plasmid pHGH207-1, having a single trp-promoter, was obtained by removal of the double lac-promoter from pHGH 207 that has a double lac-promoter followed by a single trp-promoter. This was done as follows: The trp-promoter 310bp DNA fragment was obtained from pFIF trp 69 (20) by digestion with EcoRI. This fragment was inserted into pHGH 107 (44) that had been opened with EcoRI. Thus, a plasmid was obtained (pHGH 207) that has a double lac promoter followed by the trp-promoter, flanked by EcoRI sites. The thus obtained pHGH 207 was digested with BamHI; this was partially digested with EcoRI and the largest fragment was isolated. This fragment therefore has the entire trp-promoter. From pBR322 the largest EcoRI-BamHI fragment was isolated. Both fragments were ligated and the mixture was used to transform E. coli 294. Tet^{r}, Amp^{r} colonies were isolated and most of them had the plasmid with the structure as shown for pHGH207-1. Plasmid pHGH207-1 is thus a derivative of the plasmid pHGH107 (44) and has the following properties: 1) the human growth hormone gene is flanked by the tryptophan promoter rather than the lac promoter as with pHGH107, 2) the plasmid confers ampicillin and tetracycline resistance when expressed in E. coli. (See also 47A).

20 µg of pHGH207-1 was partially digested with EcoRI and totally digested with BglII. Purification of the large vector fragment was achieved by 5 percent polyacrylamide gel electrophoresis, electroelution, phenol/CHCl₃ extraction and ethanol precipitation. 14 µg of pF1 was digested with BglII and TaqI and the 236 bp DNA fragment was isolated and purified from a 6 percent polyacrylamide gel. The following complementary DNA fragments were synthesized by the phosphotriester method (47): 
MetSerAsnGluLeuHisGlnValPro
5' AATTATGAGCAATGAATTACATCAAGTTCCAT
       TACTCGTTACTTAATGTAGTTCAAGGTAGC 5'
As indicated, the amino acid sequence Met Ser Asn Glu Leu His Gln Val Pro encodes the initiation codon, ATG, and the eight amino-terminal amino acids of high molecular weight urokinase. 50 ng of each synthetic DNA fragment were phosphorylated and the fragments were mixed, heated to 65°C for 1 minute and allowed to cool at room temperature for 5 minutes. 10 ng of the phosphorylated and mixed synthetic DNA fragments were combined with ∼200 ng of the partial EcoRI, BglII pHGH207-1 vector fragment and ∼50 ng of the 236 bp BglII TaqI DNA fragment, ligated overnight at 14°C and transformed into E. coli 294. Individual plasmid DNAs from 24 ampicillin resistant colonies were digested with EcoRI and BglII and one plasmid (pInt1) demonstrating the proper construction was selected for DNA sequence analysis. This analysis verified the correct DNA sequence through the ATG initiation codon and the amino-terminal portion of high molecular weight urokinase.

4 µg of pNCV (Section M) was digested with BglII and ClaI and the large vector fragment was isolated and purified from a 5 percent polyacrylamide gel. 30 µg of pF1 was digested with PstI and BglII and electrophoresed through a 6 percent polyacrylamide gel. The 44 bp DNA fragment was electroeluted, phenol/CHCl₃ extracted and ethanol precipitated. 4 µg of pA3 DNA were digested with PstI and TaqI and the 192 bp DNA fragment was purified from a 6 percent polyacrylamide gel. ∼100 ng of the BglII ClaI vector DNA fragment, ∼50 ng of the 192 bp fragment and ∼50 ng of the 44 bp fragment were combined and ligated overnight at 14°C then transformed into E. coli 294. BglII ClaI double digestion of plasmid DNA from several tetracycline resistant transformants demonstrated the correct construction of this new plasmid named pInt2.

5µg of pUK33trp103Ap^{R}Tc^{R} grown in E. coli GM48 (ATCC No. 39099, April 9, 1982) was digested with Bcl1 and Sal1 for isolation and purification of the 1366 bp DNA fragment. The 108 bp EcoR1-Bcl1 DNA fragment was isolated from the same plasmid. Approximately equal molar amounts of the EcoR1-Sal1 DNA fragment from pUK33trp103Ap^{R}Tc^{R} and the EcoR1-Bcl1 DNA fragment also from pUK33trp103Ap^{R}Tc^{R} were ligated overnight at 14°C to yield pInt3.

5 µg of pInt3 DNA was digested with BglII and SalI, electrophoresed through 6 percent polyacrylamide and the 1701 bp fragment containing the carboxy terminal portion of full length urokinase and the amino terminal portion of the tetracycline resistance gene was purified. ∼5 µg of p intermediate 1 DNA was digested with BglII and SalI, electrophoresed through 6 percent polyacrylamide and the large vector fragment containing the carboxy-terminal portion of the tetracycline resistance gene, the origin of replication, the ampicillin resistance gene, the trp promoter, the initiation codon ATG, and the amino-terminal portion of full length urokinase was purified. ∼100 ng of the vector fragment and ∼100 ng of the 1701 bp fragment were combined, ligated overnight at 14°C and transformed into E. coli 294. A plasmid from a tetracycline resistant and ampicillin resistant colony demonstrating the correct PvuII restriction endonuclease pattern was identified and confirmed the construction of full length urokinase downstream from the trp promoter. This is plasmid pUK54trp207-1. Full length urokinase polypeptide is produced by expression of this plasmid in E. coli 294.

### 2. Direct Expression of Pre-UK 54K in E. coli (Figure 10)

The following scheme was used to construct a plasmid for direct expression of preUK54. Two complementary DNA fragments were synthesized by the phosphotriester method (47) encoding the amino acid sequence initiation codon ATG followed by the first three N-terminal amino acids of the urokinase presequence Arg Ala Leu as shown below 
EcoR1 MetArgAlaLeu Bgl1
5' AATTATGCGTGCCCTGC
        TACGCACGGG5'
EcoR1 and Bgl1 restriction endonuclease cleavage sites flank this portion of the presequence. 50ng of each synthetic DNA fragment was phosphorylated. The phosphorylated fragments were mixed, heated to 65°C for 1 minute and allowed to cool to room temperature. 5µg of pF1 DNA was digested with Bgl1 and Bgl2 and the 310bp UK DNA fragment was isolated and purified. ∼50ng of the 310bp UK DNA fragment, ∼150ng of the partial EcoR1, BglII vector DNA fragment from pHGH207-1 (see Section N) and 10ng of the phosphorylated and mixed synthetic DNA fragments was ligated overnight at 14°C and transformed into E. coli 294. Individual plasmid DNA's isolated from several ampicillin resistant transformants were analyzed to confirm the proper construction and nucleotide sequence of the presequence of high molecular weight urokinase.

One correct plasmid was named pInt4. 5µg of pInt4 DNA was digested with BglII and EcoRv for isolation and purificaton of the vector DNA fragment containing the N-terminal nucleotides of pre-UK54, the trp promoter, ampicillin resistance genes, origin of replication and a portion of the tetracycline resistance encoding DNA. 5µg of pUK54trp207-1 DNA was digested with BglII and EcoRv. The BglIIEcoRv DNA fragment encoding the remainder of UK54 and the tetracycline resistance encoding DNA was isolated, purified and ligated with the BglII EcoRv vector DNA fragment to complete the construction of the plasmid p-preUK54trp207-1.

### P. Isolation and Characterization

Urokinase polypeptide containing residue was isolated from E. coli. The residue was dissolved in 5M guanidine HCl, containing 50 mM Tris, pH 8.0. The solution was diluted to 1M guanidine HCl, 50mM Tris HCl, pH 9, at a protein concentration of 1 ng/ml. The solution was then brought to 2 mM reduced glutathione (GSH), 0.2 mM oxidized glutathione (GSSG) and incubated overnight at room temperature. The resulting solution containing refolded polypeptide was then dialyzed into aqueous medium. The resulting solution contained urokinase polypeptide which showed 100PU/mg activity.

Upon purification following conventional techniques, the polypeptide is characterized showing the expected N-terminal sequence for both chains of the low molecular weight, bioactive material. C-terminal analysis also shows the proper sequence for both chains. The protein migrates at a molecular weight of ∼30,000 daltons. It has a specific activity of ∼170,000 PU/mg 9225,000 IU/mg), assuming 1 mg/ml has an OD₂₈₀ of 1.3.

### Q. Assays for Detection of Expression of Urokinase polypeptide

### 1. Chromogenic Substrate

### a. Theory

The assay is based on the proteolytic cleavage of a tripeptide from a chromophoric group. The rate of cleavage can be directly related to the specificity and to the concentration of the protease being tested. Urokinase cleaves the chromogenic substrate S2444 (purchased from Kabi Group Inc., Greenwich, CT). By monitoring the generation of the chromophore, one can determine the amount of functional urokinase polypeptide present in a sample. Urokinase is synthesized as an inactive precurser form, with activation occurring via the cleavage between residue 26 (lysine) and 27 (isoleucine) (numbering based on the protease clone, Figure 2A). Some preparations of urokinase polypeptide were found to have been autoactivated and/or were activated by E. coli proteases. To insure activation of urokinase polypeptide, allowing detection by this chromogenic technique, treatment of the sample with low amounts of trypsin is required. Trypsin is a protease which can cleave the lysine-isoleucine bond required for urokinase activation. However, trypsin can also cleave the chromogenic substrate, and therefore must be eliminated from the assay. Soybean trypsin inhibitor (STI) is a protein which will inactivate trypsin while having no effect on urokinase. Therefore, the assay consists of trypsin activation of urokinase polypeptide, STI inhibition of the trypsin, and, finally, addition of the chromogenic substrate to measure the functional urokinase polypeptide present.

### b. Procedure

The assay is performed as follows: 0.2mL of 0.1M Tris, pH 8.0, 50µL of the sample to be assayed, and 5µL of trypsin (0.1mg/mL in 0.1M Tris, pH 8.0 plus 0.25M CaCl₂) were added to a test tube and the sample incubated for 10 minutes at 37°C. The trypsin was inactivated by the addition of 2µL of 10mg/mL STI (in 0.1M Tris, pH 8.0). Urokinase activity was determined by adding 50µL of a 1mM solution of S2444 (in water) and incubating the reaction for 10 minutes at 37°C. Acetic acid (50µL) was added to stop the reaction, the solution centrifuged to remove a precipitate, and the absorbance at 405nm was determined. The actual amount of urokinase polypeptide can be calculated by comparison of a sample with the reading obtained by performing the assay with dilutions of a standard solution of a known amount of urokinase (obtained from Calbiochem, San Diego, CA).

### 2. Direct Assay of Plasmin Formation

### a. Theory

A much more sensitive assay for urokinase can be obtained by monitoring the urokinase catalyzed conversion of plasminogen to plasmin. Plasmin is an enzyme for which there are chromogenic substrate assays based on the same principles as described in 1 above. The basis of the assay is the determination of the amount of plasmin formed following incubation of the urokinase containing solution with a solution of plasminogen. The greater the amount of urokinase, the greater the amount of plasmin formed.

### b. Procedure

An aliquot of the sample is mixed with 0.10 ml of 0.7 mgs/ml plasminogen (in 0.5M Tris·HCl, pH 7.4, containing .012 M NaCl) and the volume adjusted to 0.15 ml. The mixture is incubated at 37°C for various times (as indicated), 0.35 ml of S2251 (1.0 mM solution in above buffer) is added and the reaction continued for 5 minutes at 37°C. Acetic acid (25 µL) is added to terminate the reaction and absorbance at 405 nm is measured. Quantitation of the amount of activity is obtained by comparison with dilutions of a standard urokinase solution.

### 3. Indirect Assay of Plasmin Formation

### a. Theory

A sensitive assay for urokinase activity has been developed (61). The assay is based on determination of plasmin formation by measuring the extent of plasmin digestion of fibrin in an agar plate containing fibrin and plasminogen. Plasmin produces a clear lysis zone in the fibrin plate. The area of this lysis zone can be correlated to the amount of urokinase in the sample.

### b. Procedure

Following the procedure of Granelli-Piperno and Reich (61), the plates were incubated one to three hours at 37°C and lysis zones measured. Quantitation was obtained by performing the assay with dilutions of a standard urokinase solution.

### R. Detection of Urokinase Activity

### 1. Bacterial Growth and Urokinase polypeptide Sample Preparation

A strain of E. coli (W3110) was transformed using a plasmid (pUK33trpLEs) containing a urokinase fusion protein. This expression vehicle (short trpLE fusion) was described above. The cells were grown on minimal media overnight, to an O.D. at 550nm of 1.2. An additional 200mL of media was added. Indole acrylic acid, a compound which appears to enhance expression of the tryptophan operon controlled genes, was added to a concentration of 10 µg/mL. The cells were incubated 2 hours and harvested. The cells obtained from 400mL of media were suspended in water and guanidine was added to a concentration of 7M (final volume of 40 mL). The solution was incubated for 90 minutes at room temperature. Insoluble material was removed by centrifugation. The supernatant was dialyzed against 0.01 M Tris·HCl, pH 7.5, containing 0.1 M NaCl for 4 hours. Insoluble material was removed by centrifugation and the sample was dialyzed for 2.5 hours against 0.01 M Tris·HCl, pH 7.5.

The sample was applied to a 3.9 x 9 cm DE-52 column, which had been equilibrated with 0.01 M Tris·HCl, pH 7.5, and the column washed with the same buffer and eluted with 0.01 M Tris·HCl, pH 7.5, containing 0.15 M NaCl. The peak of activity was pooled and used for all further studies.

### 2. Activity Detection

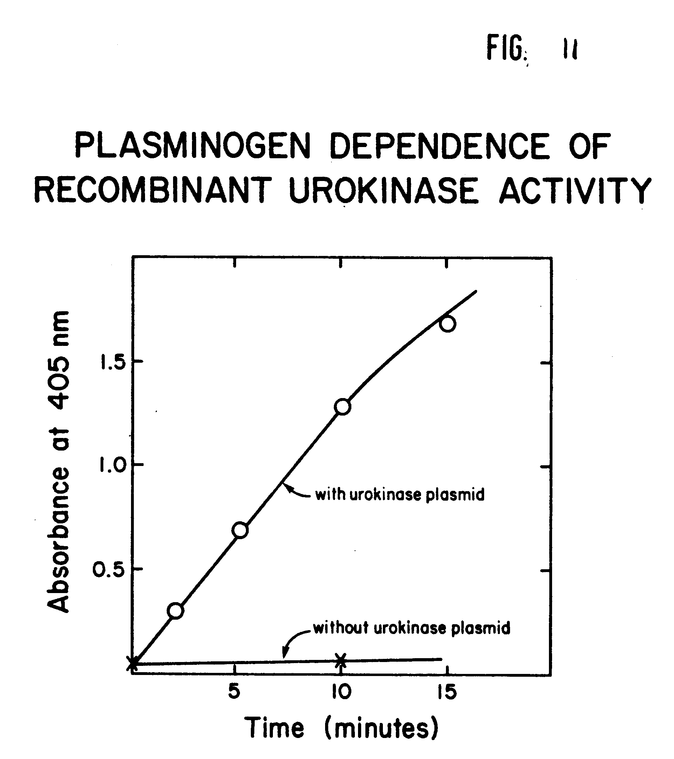

Figure 11 shows the results of the direct activation of plasminogen by fractionated E. Coli extracts when assayed under conditions similar to that described in Section Q.2. supra. An activity is generated which is dependent on the presence of plasminogen. Therefore, the activity being monitored is a plasminogen dependent activity. The activity being measured also increases with time, indicating a time dependent, catalytic generation of plasmin. These properties are consistent with those of urokinase, i.e., a catalytic activation of plasminogen. Similar extraction conditions performed on E. coli which do not contain the urokinase plasmid do not activate plasminogen under these conditions.

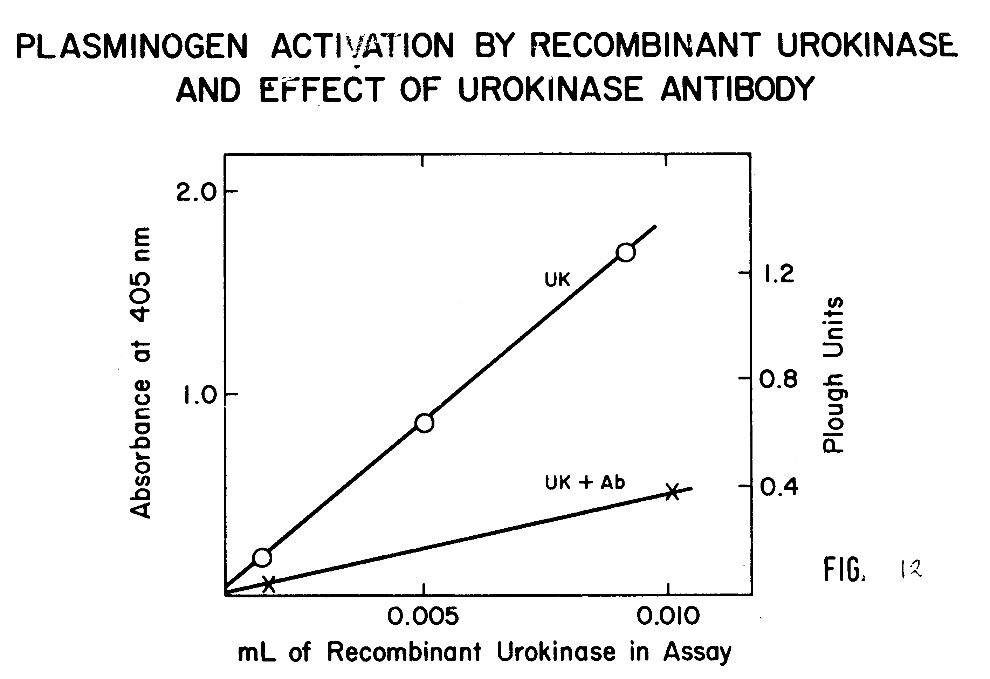

The extracts also were tested in the assays as described above in order to detect and quantitate urokinase activity. Figure 12 shows the effect of various amounts of the bacterially derived fractions in the assay described in Section Q.2 with a 10 minute activation. The values obtained are compared to those obtained using a standard urokinase solution (Plough Unitage determination). The extent of plasminogen activation is directly proportional to the amount of added cloned urokinase. Antibodies raised against purified natural urokinase are known to decrease the activity of natural urokinase. The effect of these antibodies when added to this assay at time 0 are also shown in Figure 12. A marked inhibition of the E. coli derived material is observed. This proves that the activity observed in the E. coli derived material has the same antigenic sites as natural urokinase and therefore that it is indeed urokinase polypeptide being microbially synthesized.

Similar results for activity detection and antibody inhibition are observed using the fibrin plate assay (described in Section Q.3). These results are summarized in Table I.

**TABLE I**

| FIBRIN PLATE ASSAY OF UROKINASE | | | |
|---|---|---|---|
| SAMPLE | Activity (Plough Units/mL)¹ | Activity in presence of urokinase antibody (Plough Units/mL)¹ | Percent Inhibition |
| Urokinase standard | 112 | 2.5 | 98 |
| | 11 | 0.45 | 96 |
| | 1.1 | 0 | 100 |
| Urokinase polypeptide produced herein | 480 | 1.12 | 99 |
| | 224 | 0 | 100 |

| | | | |
|---|---|---|---|
| 1 Standard curve obtained by addition of a known amount of urokinase standard to wells. Values obtained for E. coli fractions and antibody inhibition obtained by extrapolation from this standard curve. | | | |

The standard urokinase activity was inhibited 96 percent or greater by the addition of urokinase antibodies raised against this protein. Assay of the E. coli derived extracts had significant urokinase activity. This activity was almost completely inhibited when antibodies against natural urokinase were added to the assay.

The third assay (the chromogenic substrate assay) also detected a urokinase-like activity in the E. coli extracts. Since it is the least sensitive of the assays, antibody inhibition studies could not be performed due to the large quantities of antibody required to see an inhibition.

The above three assays were quantitated using the standard urokinase purchased from Calbiochem. The values obtained (Plough units per mL) were all of the same order of magnitude: 500 for fibrin plate; 100 for plasminogen activation; and 350 for the chromogenic substrate (S2444). Variations occuring are doubtless due to the relatively impure nature of the material at the time of testing.

### Pharmaceutical Compositions

The compounds of the present invention can be formulated according to known methods to prepare pharmaceutically useful compositions, whereby the human urokinase polypeptide product hereof is combined in admixture with a pharmaceutically acceptable carrier vehicle. Suitable vehicles and their formulation are described for example in Remington's Pharmaceutical Sciences by E.W. Martin. Such compositions will contain an effective amount of the polypeptide hereof together with a suitable amount of vehicle in order to prepare pharmaceutically acceptable compositions suitable for effective administration to the host.

### A. Parenteral Administration

The human urokinase polypeptide hereof may be parenterally administered to subjects suffering from thromboembolic diseases or conditions. Dosage and dose rate may parallel those currently in use in clinical applications of other cardiovascular, thrombolytic agents, e.g., about 4400 IU/kg body weight as an intravenous priming dose followed by a continuous intravenous infusion at about 4400 IU/kg/hr. for 12 hours, in patients suffering from pulmonary embolism.

As one example of an appropriate dosage form for essentially homogeneous human urokinase polypeptide in parenteral form applicable herein, a vial containing 250000 IU urokinase activity, 25 mg. mannitol and 45 mg. sodium chloride, may be reconstituted with 5 ml sterile water for injection and admixed with a suitable volume of 0.9 percent Sodium Chloride Injection or 5 percent Dextrose Injection for intravenous administration.

The human urokinase polypeptide hereof has been defined by means of determined DNA gene and deductive amino acid sequencing. It will be understood that natural allelic variations exist and occur from individual to individual. These variations may be demonstrated by (an) amino acid difference(s) in the overall sequence or by deletions, substitutions, insertions, inversions or additions of (an) amino acid(s) in said sequence. In addition, the potential exists in the use of recombinant DNA technology for the preparation of various human urokinase polypeptide derivatives, variously modified by resultant single or multiple amino acid substitutions, deletions, additions or replacements, for example, by means of site directed mutagenesis of the underlying DNA. All such modifications and allelic variations resulting in derivatives of human urokinase polypeptide are included within the ambit of this invention so long as the essential, characteristic human urokinase activity remains unaffected in kind.

Notwithstanding that reference has been made to particular preferred embodiments, it will be further understood that the present invention is not to be construed as limited to such, rather to the lawful scope of the appended claims.

### Bibliography

- 1.: United States Patent No. 3355361.
- 2.: United States Patent No. 3926727.
- 3.: United States Patent No. 4029767.
- 4.: United States Patent No. 4258030.
- 5.: United States Patent No. 4271150.
- 6.: European Patent Application Publn. No. 0037687.
- 7.: United States Patent No. 3555000.
- 8.: Wallen, P., Proc. Serono Symp. 9, 91 (1977).
- 9.: Thorsen, S., et al., Thrombos. Diathes. haemorrh. 28, 65 (1972).
- 9A.: Barnett and Baron, Proc. Soc. Exptl. Biol. 102, 308 (1959).
- 9B.: Banlow and Lazer, Thrombosis Res. 1, 201 (1972).
- 10.: Husain, S.S., et al., Thromb. Hemostas 46, 11 (1981).
- 10A.: Wun et al., J. Biol. Chem. 257, 3276 (1982).
- 11.: Ratzkin et al., Proc. Natl. Acad. Sci. (USA) 78, 3313 (1981).
- 11A.: Bollen, A. et al., Biochem. Biophys. Res. Commun. 103, 391 (1981).
- 12.: British Patent Application Publn. No. 2007676A.
- 13.: Wetzel, American Scientist 68, 664 (1980).
- 14.: Microbiology, 2d Ed., Harper and Row Publications, Inc., Hagerstown, Maryland (1973), esp. pp. 1122 et seq.
- 15.: Scientific American 245, 66 et seq. (1981).
- 16.: British Patent Application PubIn. No. 2055382A.
- 17.: German Offenlegungsschrift 2644432.
- 18.: Chang et al., Nature 275, 617 (1978).
- 19.: Itakura et al., Science 198, 1056 (1977).
- 20.: Goeddel et al., Nucleic Acids Research 8, 4057 (1980).
- 21.: European Patent Application Publn. No. 0036776.
- 22.: Siebenlist et al., Cell 20, 269 (1980).
- 37.: Bolivar et al., Gene 2, 95 (1977).
- 38.: Vetterlein et al., J. Biol. Chem. 255, 3665 (1980).
- 39.: Eagle, H., Science 130, 432 (1959).
- 40.: Lynch et al, Virology 98, 251 (1979).
- 41.: Aviv et al., Proc. Natl. Acad. Sci. USA 69, 1408 (1972).
- 42.: Lehrach et al., Biochemistry 16, 4743 (1977).
- 43.: Jackson et al., Proc. Natl. Acad. Sci. (USA) 74, 5598 (1977).
- 44.: Goeddel et al., Nature 281, 544 (1979).
- 45.: Wickens et al., J. Biol. Chem. 253, 2433 (1978).
- 46.: Chang et al., Nature 275, 617 (1978).
- 47.: Crea et al., Proc. Natt. Acad. Sci. (USA) 75, 5765 (1978).
- 47A.: European Patent Application Publication No. 0036776.
- 48.: Miller, Experiments in Molecular Genetics, p. 431-3, Cold Spring Harbor Lab., Cold Spring Harbor, New York (1972).
- 49.: Grunstein et al., Proc. Natl. Acad. Sci. U.S.A. 72, 3961 (1975).
- 50.: Wallace et al., Nucleic Acids Research 9, 879 (1981).
- 51.: Birnboim et al., Nucleic Acids Research 7, 1513 (1979).
- 52.: Smith, Methods Enzymot. 65, 560 (1980).
- 53.: Messing et al., Nucleic Acids Res. 9, 309 (1981).
- 54.: Taylor et al., Biochem. Biophys. Acta 442, 324 (1976).
- 55.: Fritsch et al., Cell 19, 959 (1980).
- 56.: Goeddel et al., Nature 290, 20 (1981).
- 57.: Blobel et al., Biomembranes, Vol. 2, ed. Manson, pp. 193, Plenum.
- 58.: Goeddel et al., Nature 287, 411 (1980).
- 59.: Itakura et al., Science 198, 1056 (1977).
- 60.: Bingham et al., Nucleic Acids Research 5, 3457 (1978).
- 61.: Granelli-Piperino and Reich, J. Exp. Med. 148, 223.

## Claims (Claims for the following Contracting State(s): BE, CH, DE, FR, GB, IT, LU, NL, SE)

1. An unglycosylated polypeptide having an intact lysine - isoleucine bond at the position corresponding to amino acids 158-159 in the native prourokinase molecule, and having sufficient of the amino acid sequence set out in Figures 4A and 4B such that, on activation, the activated polypeptide exhibits the enzymatic activity of human urokinase.

2. A polypeptide according to claim 1 which is unglycosylated met-prourokinase protein.

3. A polypeptide according to claim 1 which is unglycosylated prourokinase protein.

4. A polypeptide according to any one of the preceding claims having the sequence Lys-Ile-Ile-Gly-Gly, at positions corresponding to amino acids 158-162 in the native prourokinase molecule.

5. A polypeptide according to any one of the preceding claims having the amino acid sequence or substantially the amino acid sequence as depicted in Figs 4A and 4B.

6. An isolated DNA sequence encoding a polypeptide according to any one of the preceding claims.

7. A DNA sequence according to claim 6 operably linked with a DNA sequence capable of effecting expression thereof.

8. A replicable expression vehicle capable, in a transformant microorganism of expressing a DNA sequence according to claim 6 or 7.

9. A microorganism transformed with a vehicle according to claim 8.

10. A microorganism according to claim 9 which is a transformed strain of E. coli.

11. A composition comprising a therapeutically effective amount of a polypeptide according to any one of claims 1 to 5 in admixture with a pharmaceutically acceptable carrier.

12. A polypeptide according to any one of claims 1 to 5 for use in a method of treatment of the human or animal body for the treatment of vascular diseases.

13. A process for producing a polypeptide as defined in any one of claims 1-5 which comprises expressing DNA sequence according to claim 6 or 7 to obtain the unglycosylated polypeptide that, on activation, exhibits the enzymatic activity of human urokinase.

14. A process according to claim 13 in which the resulting activatable polypeptide is converted by proteolytic cleavage to an enzymatically active polypeptide.

## Claims (Claims for the following Contracting State(s): AT)

1. A process for producing an expression vehicle which comprises operably linking a first DNA sequence to a second DNA sequence capable of effecting expression of said first DNA sequence in a selected host cell to produce an unglycosylated prourokinase, wherein said first DNA sequence encodes a polypeptide having an intact lysine - isoleucine bond at the position corresponding to amino acids 158-159 in the native prourokinase molecule, having sufficient of the amino acid sequence set out in Figures 4A and 4B, said expressed polypeptide being such that, on activation, the activated polypeptide exhibits the enzymatic activity of human urokinase.

2. A process according to claim 1 wherein the expressed polypeptide is unglycosylated met-prourokinase.

3. A process according to claim 1 or 2 wherein the polypeptide expressed is one having the sequence Lys-Ile-Ile-Gly-Gly, at positions corresponding to amino acids 158-162 in the native prourokinase molecule.

4. A process according to any one of the preceding claims wherein the expressed polypeptide is one having the amino acid sequence or substantially the amino acid sequence as depicted in Figs 4A and 4B.

5. A process according to any one of the preceding claims wherein the resulting expression vehicle is capable, in a transformant microorganism, of expressing the first DNA sequence in a selected host cell.

6. A process for producing a microorganism capable of expressing an unglycosylated polypeptide as defined in claim 1 which comprises transforming a microorganism with an expression vehicle produced by a process according to any one of the preceding claims.

7. A process according to claim 6 wherein the microorganism is E. coli.

8. A process for producing an unglycosylated polypeptide which comprises cultivating a microbial host harboring an expression vehicle obtained by a process according to any one of claims 1-4.

9. A process according to claim 8 wherein the host microorganism is E. coli.

10. A process according to claim 8 or 9 in which the resulting activatable polypeptide is converted by proteolytic cleavage to an enzymatically active polypeptide.

## Patentansprüche (Patentansprüche für folgende(n) Vertragsstaat(en): BE, CH, DE, FR, GB, IT, LU, NL, SE)

1. Unglykosyliertes Polypeptid mit einer intakten Lysin-Isoleucin-Bindung an der Position, die den Aminosäuren 158-159 im nativen Prourokinase-Molekül entspricht, und das genügend von der in den Figuren 4A und 4B dargestellten Aminosäuresequenz aufweist, so daß, bei Aktivierung, das aktivierte Polypeptid die enzymatische Aktivität von Humanurokinase zeigt.

2. Polypeptid nach Anspruch 1, dadurch **gekennzeichnet**, daß es unglykosyliertes Met-Prourokinase-Protein ist.

3. Polypeptid nach Anspruch 1, dadurch **gekennzeichnet**, daß es unglykosyliertes Prourokinase-Protein ist.

4. Polypeptid nach einem der vorstehenden Ansprüche, dadurch **gekennzeichnet**, daß es die Sequenz Lys-Ile-Ile-Gly-Gly an den Positionen, die den Aminosäuren 158-162 im nativen Prourokinase-Molekül entsprechen, aufweist.

5. Polypeptid nach einem der vorstehenden Ansprüche mit der Aminosäuresequenz oder im wesentlichen der Aminosäuresequenz, wie in den Figuren 4A und 4B dargestellt.

6. Isolierte DNA-Sequenz, die für ein Polypeptid nach einem der vorstehenden Ansprüche codiert.

7. DNA-Sequenz nach Anspruch 6, dadurch **gekennzeichnet**, daß sie mit einer DNA-Sequenz, die in der Lage ist, deren Expression zu bewirken, funktionsfähig verknüpft ist.

8. Replizierbares Expressionsvehikel, dadurch **gekennzeichnet**, daß es in der Lage ist, in einem transformanten Mikroorganismus eine DNA-Sequenz nach Anspruch 6 oder 7 zu exprimieren.

9. Mikroorganismus, dadurch **gekennzeichnet**, daß er mit einem Vehikel nach Anspruch 8 transformiert ist.

10. Mikroorganismus nach Anspruch 9, dadurch **gekennzeichnet**, daß er ein transformierter Stamm von E. coli ist.

11. Präparat, dadurch **gekennzeichnet**, daß es eine therapeutisch wirksame Menge eines Polypeptids nach einem der Ansprüche 1 bis 5 im Gemisch mit einem pharmazeutisch annehmbaren Träger enthält.

12. Polypeptid nach einem der Ansprüche 1 bis 5 zur Verwendung bei einem Behandlungsverfahren des menschlichen oder tierischen Körpers für die Behandlung von Gefäßerkrankungen.

13. Verfahren zur Herstellung eines in einem der Ansprüche 1 bis 5 definierten Polypeptids, gekennzeichnet durch die Expression einer DNA-Sequenz nach Anspruch 6 oder 7, um das unglykosylierte Polypeptid zu erhalten, das, bei Aktivierung, die enzymatische Aktivität von Humanurokinase zeigt.

14. Verfahren nach Anspruch 13, dadurch **gekennzeichnet**, daß das resultierende aktivierbare Polypeptid durch proteolytische Spaltung in ein enzymatisch aktives Polypeptid umgewandelt wird.

## Patentansprüche (Patentansprüche für folgende(n) Vertragsstaat(en): AT)

1. Verfahren zur Herstellung eines Expressionsvehikels, gekennzeichnet durch die funktionsfähige Verknüpfung einer ersten DNA-Sequenz mit einer zweiten DNA-Sequenz, die in der Lage ist, die Expression der ersten DNA-Sequenz in einer selektierten Wirtszelle zu bewirken, um eine unglykosylierte Prourokinase herzustellen, wobei die erste DNA-Sequenz für ein Polypeptid mit einer intakten Lysin-Isoleucin-Bindung an der Position, die den Aminosäuren 158-159 im nativen Prourokinase-Molekül entspricht, codiert, welches genügend von der in den Figuren 4A und 4B dargestellten Aminosäuresequenz aufweist, so daß das exprimierte Polypeptid so beschaffen ist, daß bei Aktivierung das aktivierte Polypeptid die enzymatische Aktivität von Humanurokinase zeigt.

2. Verfahren nach Anspruch 1, dadurch **gekennzeichnet**, daß das exprimierte Polypeptid unglykosylierte Met-Prourokinase ist.

3. Verfahren nach Anspruch 1 oder 2, dadurch **gekennzeichnet**, daß das exprimierte Polypeptid ein solches ist, das die Sequenz Lys-Ile-Ile-Gly-Gly an Positionen aufweist, die den Aminosäuren 158-162 im nativen Prourokinase-Molekül entsprechen.

4. Verfahren nach einem der vorstehenden Ansprüche, dadurch **gekennzeichnet**, daß das exprimierte Polypeptid ein solches ist, das die Aminosäuresequenz oder im wesentlichen die Aminosäuresequenz, wie in den Figuren 4A und 4B dargestellt, aufweist.

5. Verfahren nach einem der vorstehenden Ansprüche, dadurch **gekennzeichnet**, daß das resultierende Expressionsvehikel in der Lage ist, in einem transformanten Mikroorganismus die erste DNA-Sequenz in einer selektierten Wirtszelle zu exprimieren.

6. Verfahren zur Herstellung eines Mikroorganismus, der in der Lage ist, ein unglykosyliertes Polypeptid, wie in Anspruch 1 definiert, zu exprimieren, gekennzeichnet durch das Transformieren eines Mikroorganismus mit einem Expressionsvehikel, hergestellt durch ein Verfahren nach einem der vorstehenden Ansprüche.

7. Verfahren nach Anspruch 6, dadurch **gekennzeichnet**, daß der Mikroorganismus E. coli ist.

8. Verfahren zur Herstellung eines unglykosylierten Polypeptids, gekennzeichnet durch das Kultivieren eines mikrobiellen Wirts, der ein Expressionsvehikel enthält, das nach einem Verfahren nach einem der Ansprüche 1 bis 4 erhalten wurde.

9. Verfahren nach Anspruch 8, dadurch **gekennzeichnet**, daß der Wirtsmikroorganismus E. coli ist.

10. Verfahren nach Anspruch 8 oder 9, dadurch **gekennzeichnet**, daß das resultierende aktivierbare Polypeptid durch proteolytische Spaltung in ein enzymatisch aktives Polypeptid umgewandelt wird.

## Revendications (Revendications pour l'(les) Etat(s) contractant(s) suivant(s): BE, CH, DE, FR, GB, IT, LU, NL, SE)

1. Polypeptide non glycosylé ayant une liaison lysine-isoleucine intacte en la position correspondant aux amino-acides 158-159 dans la molécule de prourokinase naturelle, et ayant une partie suffisante de la séquence d'amino-acides indiquée sur les figures 4A et 4B pour que, par activation, le polypeptide activé présente l'activité enzymatique de l'urokinase humaine.

2. Polypeptide suivant la revendication 1, qui consiste en met-prourokinase non glycosylée.

3. Polypeptide suivant la revendication 1, qui consiste en prourokinase non glycosylée.

4. Polypeptide suivant l'une quelconque des revendications précédentes, ayant la séquence Lys-Ile-Ile-Gly-Gly en les positions correspondant aux amino-acides 158-162 dans la molécule de prourokinase naturelle.

5. Polypeptide suivant l'une quelconque des revendications précédentes, ayant la séquence d'amino-acides ou pratiquement la séquence d'amino-acides représentée sur les figures 4A et 4B.

6. Séquence d'ADN isolée codant pour un polypeptide suivant. l'une quelconque des revendications précédentes.

7. Séquence d'ADN suivant la revendication 6, liée de manière fonctionnelle à une séquence d'ADN capable d'en effectuer l'expression.

8. Véhicule d'expression réplicable capable, dans un micro-organisme transformant, d'exprimer une séquence d'ADN suivant la revendication 6 ou 7.

9. Micro-organisme transformé avec un véhicule suivant la revendication 8.

10. Micro-organisme suivant la revendication 9, qui consiste en une souche transformée de E. coli.

11. Composition comprenant une quantité à effet thérapeutique d'un polypeptide suivant l'une quelconque des revendications 1 à 5 en mélange avec un support pharmaceutiquement acceptable.

12. Polypeptide suivant l'une quelconque des revendications 1 à 5, destiné à être utilisé dans une méthode de traitement de l'organisme de l'homme ou d'un animal pour le traitement de maladies vasculaires.

13. Procédé de production d'un polypeptide tel que défini dans l'une quelconque des revendications 1 à 5, qui comprend l'expression d'une séquence d'ADN suivant la revendication 6 ou 7 pour obtenir le polypeptide non glycosylé qui, par activation, présente l'activité enzymatique de l'urokinase humaine.

14. Procédé suivant la revendication 13, dans lequel le polypeptide activable résultant est transformé par clivage protéolytique en un polypeptide enzymatiquement actif.

## Revendications (Revendications pour l'(les) Etat(s) contractant(s) suivant(s): AT)

1. Procédé de production d'un véhicule d'expression, qui comprend la liaison de manière fonctionnelle d'une première séquence d'ADN à une seconde séquence d'ADN capable d'effectuer l'expression de ladite première séquence d'ADN dans une cellule-hôte choisie pour produire une prourokinase non glycosylée, dans lequel ladite première séquence d'ADN code pour un polypeptide ayant une liaison lysine-isoleucine intacte en la position correspondant aux amino-acides 158-159 dans la molécule de prourokinase naturelle, ayant une partie suffisante de la séquence d'amino-acides représentée sur les figures 4A et 4B, ledit polypeptide exprimé étant tel que, par activation, le polypeptide activé présente l'activité enzymatique de l'urokinase humaine.

2. Procédé suivant la revendication 1, dans lequel le polypeptide exprimé consiste en met-prourokinase non glycosylée.

3. Procédé suivant la revendication 1 ou 2, dans lequel le polypeptide exprimé est un polypeptide ayant la séquence Lys-Ile-Ile-Gly-Gly en les positions correspondant aux amino-acides 158-162 dans la molécule de prourokinase naturelle.

4. Procédé suivant l'une quelconque des revendications précédentes, dans lequel le polypeptide exprimé est un polypeptide ayant la séquence d'amino-acides ou pratiquement la séquence d'amino-acides représentée sur les figures 4A et 4B.

5. Procédé suivant l'une quelconque des revendications précédentes, dans lequel le véhicule d'expression résultant est capable, dans un micro-organisme transformant, d'exprimer la première séquence d'ADN dans une cellule-hôte choisie.

6. Procédé de production d'un micro-organisme capable d'exprimer un polypeptide non glycosylé tel que défini dans la revendication 1, qui comprend la transformation d'un micro-organisme avec un véhicule d'expression produit par un procédé suivant l'une quelconque des revendications précédentes.

7. Procédé suivant la revendication 6, dans lequel le micro-organisme est E. coli.

8. Procédé de production d'un polypeptide non glycosylé, qui comprend la culture d'un hôte microbien renfermant un véhicule d'expression obtenu par un procédé suivant l'une quelconque des revendications 1 à 4.

9. Procédé suivant la revendication 8, dans lequel le micro-organisme hôte est E. coli.

10. Procédé suivant la revendication 8 ou 9, dans lequel le polypeptide activable résultant est transformé par clivage protéolytique en un polypeptide enzymatiquement actif.
